(19) Europäisches Patentamt
European Patent Office
Office européen des brevets

(11) EP 3 337 447 B2

(12) NEW EUROPEAN PATENT SPECIFICATION
After opposition procedure

(45) Date of publication and mention of the opposition decision:
**07.06.2023 Bulletin 2023/23**

(45) Mention of the grant of the patent:
**15.07.2020 Bulletin 2020/29**

(21) Application number: **16753382.7**

(22) Date of filing: **17.08.2016**

(51) International Patent Classification (IPC):
***A61K 8/41*** *(2006.01)* ***A61Q 5/00*** *(2006.01)*
***A61K 8/58*** *(2006.01)* ***A61Q 5/06*** *(2006.01)*
***A61K 8/49*** *(2006.01)*

(52) Cooperative Patent Classification (CPC):
**A61K 8/4913; A61K 8/585; A61Q 5/002;**
**A61Q 5/06;** A61K 2800/882; A61K 2800/884

(86) International application number:
**PCT/EP2016/069535**

(87) International publication number:
**WO 2017/029336 (23.02.2017 Gazette 2017/08)**

# (54) PROCESS FOR TREATING KERATIN FIBRES WITH AN ALKOXYSILANE POLYMER BEARING A NUCLEOPHILIC GROUP AND AN ACTIVATED (THIO)ESTER

VERFAHREN ZUR BEHANDLUNG VON KERATINFASERN MIT EINEM ALKOXYSILANPOLYMER MIT NUKLEOPHILER GRUPPE UND EINEM AKTIVIERTEN (THIO)ESTER

PROCÉDÉ DE TRAITEMENT DE FIBRES DE KÉRATINE AVEC UN POLYMÈRE D’ALCOXYSILANE PORTANT UN GROUPE NUCLÉOPHILE ET UN (THIO)ESTER ACTIVÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.08.2015 FR 1557769**

(43) Date of publication of application:
**27.06.2018 Bulletin 2018/26**

(73) Proprietor: **L’OREAL**
**75008 Paris (FR)**

(72) Inventors:
- **BAGHDADLI, Nawel**
  **91300 Massy (FR)**
- **JEGOU, Gwenaëlle**
  **91240 Saint Michel sur Orge (FR)**

(74) Representative: **L’Oreal**
**Service D.I.P.I.**
**9, rue Pierre Dreyfus**
**92110 Clichy (FR)**

(56) References cited:
**EP-A2- 2 111 848**
**EP-A2- 2 343 041**
**WO-A1-2014/095821**
**WO-A1-2015/075236**
**WO-A1-2015/128566**
**WO-A2-2008/156327**
**WO-A2-2012/163868**
**WO-A2-2012/163869**
**FR-A1- 2 982 155**
**JP-A- 2012 240 983**
**KR-A- 20130 114 468**
**KR-A- 20130 114 468**
**US-A- 4 344 763**
**US-A1- 2009 293 899**
**US-A1- 2010 275 387**

EP 3 337 447 B2

## Description

The present invention relates to a process for treating keratin fibres, comprising the application to the fibres i) of at least one nucleophilic alkoxysilane polymer, in particular at least one aminoalkoxysilane polymer, and ii) at least one aliphatic-chain activated (thio)ester. The invention also relates to a cosmetic composition comprising ingredients i) and ii) and to a kit comprising ingredients i) and ii) for performing such a process.

Hair is generally damaged and embrittled by the action of chemical treatments such as dyeing, bleaching, permanent-waving, relaxing and repeated washing.

Is known that certain hair treatments such as dyeing, bleaching, permanent-waving and relaxing can attack the hair fibre and give rise especially to the loss of some of the constituents thereof that are present in the natural state, in particular fatty acids such as 18-methyleicosanoic acid. The hair is thereby damaged and becomes sensitive.

The damaged fibre has an electrostatic nature, making it difficult to correctly shape the hair, especially during combing or brushing. Furthermore, the damaged fibre has a more hydrophilic nature, making it very sensitive to water: the fibre has a tendency to swell and to frizz on contact with ambient moisture, and under these conditions does not ensure good hold of the hairstyle.

It is known that the cosmetic qualities of the hair may be improved by applying various compositions based on active agents or polymers for imparting various properties thereto, such as sheen, ease of disentangling, body, suppleness, liveliness or softness. To obtain good efficacy, these active agents should, of course, have a certain affinity for keratin fibres.

WO 2008/156 327 discloses a composition comprising lipids bearing functional groups, which, after application to the hair or the skin, are covalently bonded to the surface of the keratin materials. The functional groups are, for example, hydroxysuccinimidyl ester groups. The lipids, for their part, are $C_8$-$C_{28}$ fatty acids. However, the hydrophobicity effect obtained at the surface of the hair fades out in the course of successive shampoo washes and does not show satisfactory persistence on shampooing.

Moreover, the application of treating polymers to the hair may be harmful to the maintenance of a good feel, especially over the course of successive applications: the treated hair is charged, feels coarse, is not smooth, is grating and is sparingly cosmetic.

WO 2008/1563 and KR 2012-0036877 also disclose processes for treating the hair by applying an activated ester.

Nevertheless, the cosmetic properties of hair treated with these processes are not always satisfactory, especially in terms of hydrophobicity, which may have the effect of increasing the frizziness and reducing the ease of styling and/or the control of the hair volume. These treatments therefore do not make it possible to obtain optimum hydrophobicity and persistence properties. There is thus a need for a hair treatment process that makes it possible to obtain improved hydrophobicity and persistence properties.

Moreover, it is known practice to use aminoalkoxysilanes in hair treatment, as disclosed in US 4 344 763 and US 2009/293899. JP 2012/240 983 describes N-succinimide esters bonded to a silane via an amide function, used for immobilizing proteins as a biosensor substrate. On the other hand document FR 2 982 155 discloses the use of nucleophilic alkoxysilane for treating hair.

The object of the present invention is to propose a cosmetic process for treating keratin fibres, especially the hair, making it possible to restore to a damaged fibre the surface physicochemical properties of a natural fibre, and to do so in a long-lasting manner.

A subject of the invention is thus a process for treating keratin materials, comprising:

i) application to the keratin fibres of a cosmetic composition (A) comprising at least one nucleophilic alkoxysilane polymer or oligomer, in which the nucleophilic alkoxysilane polymer(s) or oligomer is derived from a monomer of formula **(I)** below, and also the acid salts thereof, and the solvates thereof such as hydrates:

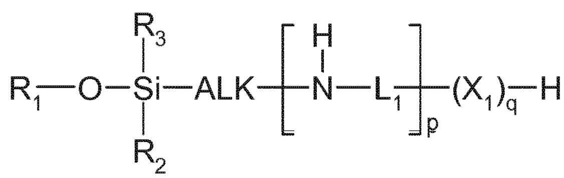

**(I)**

in which formula **(I):**

- **p** and **q,** which may be identical or different, are equal to 0 or 1, with the sum p + q being greater than or equal

to 1, preferably p + q is equal to 1 or 2, in particular p is 0 and q is 1;

- **$X_1$** represents an amino group -N($R_a$)- with $R_a$ representing a hydrogen atom or a group chosen from ($C_1$-$C_8$)alkyl such as methyl, ($C_5$-$C_7$)cycloalkyl such as cyclohexyl, aryl such as phenyl, and aryl($C_1$-$C_4$)alkyl such as benzyl, preferably -N(H)-;
- **ALK** represents a linear or branched $C_1$-$C_{10}$, in particular linear, saturated divalent hydrocarbon-based chain, more particularly $-(CH_2)_x-$ with x representing an integer between 1 and 6 inclusive, preferably between 1 and 4 such as methylene, ethylene or propylene; more preferentially methylene or propylene;
- **$R_1$** represents a hydrogen atom or a ($C_1$-$C_6$)alkyl group such as methyl or ethyl;
- **$R_2$** and **$R_3$,** which may be identical or different, preferably identical, represent a group chosen from hydroxyl, ($C_1$-$C_6$)alkyl and ($C_1$-$C_6$)alkoxy, in particular $C_1$-$C_4$ alkoxy such as ethoxy;
- **$L_1$** represents a linear or branched saturated $C_1$-$C_{20}$ divalent hydrocarbon-based chain; in particular linear, more particularly $-(CH_2)_x-$ with x as defined previously; in particular, $L_1$ represents an ethylene or propylene group; and

ii) application of a cosmetic composition (B) comprising at least one activated (thio)ester compound;

it being understood that compositions (A) and (B) may be applied to the keratin fibres together or separately, preferably separately.

Another subject of the invention is a cosmetic composition (C), as claimed in claim 15.

The treatment process is in particular a process for caring for keratin fibres, in particular human keratin fibres such as the hair.

A subject of the invention is also a multi-compartment device or kit, as claimed in claim 16.

Ingredients i) and ii) of the kit may be in cosmetic compositions (A) and (B) as defined previously. The form of the compositions is known and adapted to be stored (cans, tube, spray can or aerosol can especially).

Such a kit allows the process for treating keratin materials according to the invention to be performed.

The process and the composition according to the invention allow the damaged fibre to regain a hydrophobic surface state close to that of natural hair, and to do so in a long-lasting manner, the hydrophobicity effect being persistent after one or more shampoo washes performed on the treated keratin fibres. The general appearance of the hair is improved, the treated hair is less electrostatic, has less body and less frizziness on contact with ambient moisture, thus contributing to good shaping of the hair, in particular of fine hair. The treated hair also has a good, soft, non-coarse cosmetic feel.

In particular, as shown by the examples, the process and the composition according to the invention make it possible to obtain improved hydrophobicity and persistence properties, while at the same time having a good feel. Application solely of the activated (thio)ester to the hair does not make it possible to achieve the optimum hydrophobicity property, and application solely of the nucleophilic alkoxysilane polymer gives a charged, coarse and thus sparingly cosmetic feel.

For the purposes of the present invention and unless otherwise indicated:

- The term *"oligomer"* means a compound comprising from 2 to 4 repeating monomer units;
- The term *"polymer"* means a compound comprising at least 5 monomer units and preferably between 5 and 50 000 repeating monomer units;
- the "*aryl*" or "*heteroaryl*" radicals or the aryl or heteroaryl part of a radical may be substituted with at least one substituent borne by a carbon atom, chosen from:

  - a $C_1$-$C_8$ alkyl radical, optionally substituted with one or more radicals chosen from hydroxyl, $C_1$-$C_2$ alkoxy, (poly)hydroxy($C_2$-$C_4$)alkoxy, acylamino, amino substituted with two alkyl radicals;
  - a halogen atom;
  - a hydroxyl group;
  - a $C_1$-$C_2$ alkoxy radical;
  - a (poly)hydroxy($C_2$-$C_4$)alkoxy radical;
  - an amino radical;
  - a 5- or 6-membered heterocycloalkyl radical;
  - a 5- or 6-membered heteroaryl radical, optionally substituted with a ($C_1$-$C_4$)alkyl radical, preferentially methyl;
  - an amino radical substituted with one or two identical or different alkyl radicals;
  - an acylamino radical (-NR-C(O)-R') in which the radical R is a hydrogen atom or a $C_1$-$C_4$ alkyl radical optionally bearing at least one hydroxyl group and the radical R' is a $C_1$-$C_2$ alkyl radical;
  - a carbamoyl radical ($(R)_2N$-C(O)-) in which the radicals R, which may be identical or different, represent a hydrogen atom or a $C_1$-$C_4$ alkyl radical optionally bearing at least one hydroxyl group;

- an alkylsulfonylamino radical (R'-$S(O)_2$-N(R)-) in which the radical R represents a hydrogen atom or a $C_1$-$C_4$ alkyl radical optionally bearing at least one hydroxyl group and the radical R' represents a $C_1$-$C_4$ alkyl radical or a phenyl radical; an aminosulfonyl radical ($(R)_2N$-$S(O)_2$-) in which the radicals R, which may be identical or different, represent a hydrogen atom or a $C_1$-$C_4$ alkyl radical optionally bearing at least one hydroxyl group;
- a carboxyl radical in the acid or salified form (preferably salified with an alkali metal or a substituted or unsubstituted ammonium);
- a cyano group;
- a nitro or nitroso group;
- a polyhaloalkyl group, preferentially the trifluoromethyl group;

the cyclic or heterocyclic part of a non-aromatic radical may be substituted with at least one halogen atom or a substituent chosen from the following groups:

- hydroxyl;
- $C_1$-$C_4$ alkoxy, $C_2$-$C_4$ (poly)hydroxyalkoxy;
- $C_1$-$C_4$ alkyl;
- alkylcarbonylamino (R-C(O)-N(R')-) in which the radical R' is a hydrogen atom or a $C_1$-$C_4$ alkyl radical optionally bearing at least one hydroxyl group, and the radical R is a $C_1$-$C_2$ alkyl radical or an amino radical optionally substituted with one or two $C_1$-$C_4$ alkyl groups, which may be identical or different, optionally bearing at least one hydroxyl group, said alkyl radicals possibly forming, with the nitrogen atom to which they are attached a saturated or unsaturated, optionally substituted 5- to 7-membered heterocycle optionally comprising at least one other nitrogen or non-nitrogen heteroatom;
- alkylcarbonyloxy (R-C(O)-O-) in which the radical R is a $C_1$-$C_4$ alkyl radical or an amino group optionally substituted with one or two identical or different $C_1$-$C_4$ alkyl groups optionally bearing at least one hydroxyl group, said alkyl radicals possibly forming with the nitrogen atom to which they are attached a saturated or unsaturated, optionally substituted 5- to 7-membered heterocycle, optionally comprising at least one other nitrogen or non-nitrogen heteroatom;
- alkoxycarbonyl (R-G-C(O)-) in which the radical R is a $C_1$-$C_4$ alkoxy radical, G is an oxygen atom or an amino group optionally substituted with a $C_1$-$C_4$ alkyl group optionally bearing at least one hydroxyl group, said alkyl radical possibly forming with the nitrogen atom to which they are attached a saturated or unsaturated, optionally substituted 5- to 7-membered heterocycle, optionally comprising at least one other nitrogen or non-nitrogen heteroatom;

- ▪ a cycloalkyl, cycloalkenyl, heterocyclic or heterocycloalkyl radical, or a non-aromatic part of an aryl or heteroaryl radical, may also be substituted with one or more oxo groups;
- ▪ a hydrocarbon-based chain is unsaturated when it comprises one or more double bonds and/or one or more triple bonds;
- ▪ an *aryl* radical represents a monocyclic or fused or non-fused polycyclic carbon-based group comprising from 6 to 22 carbon atoms, and in which at least one ring is aromatic; preferentially, the aryl radical is a phenyl, biphenyl, naphthyl, indenyl, anthracenyl or tetrahydronaphthyl;
- ▪ a *heteroaryl radical* represents an optionally cationic, 5- to 22-membered, monocyclic or fused or non-fused polycyclic group, comprising from 1 to 6 heteroatoms chosen from nitrogen, oxygen, sulfur and selenium, at least one ring of which is aromatic; preferentially, a heteroaryl radical is chosen from acridinyl, benzimidazolyl, benzobistriazolyl, benzopyrazolyl, benzopyridazinyl, benzoquinolyl, benzothiazolyl, benzotriazolyl, benzoxazolyl, pyridinyl, tetrazolyl, dihydrothiazolyl, imidazopyridyl, imidazolyl, indolyl, isoquinolyl, naphthoimidazolyl, naphthoxazolyl, naphthopyrazolyl, oxadiazolyl, oxazolyl, oxazolopyridyl, phenazinyl, phenoxazolyl, pyrazinyl, pyrazolyl, pyrilyl, pyrazoyltriazyl, pyridyl, pyridinoimidazolyl, pyrrolyl, quinolyl, tetrazolyl, thiadiazolyl, thiazolyl, thiazolopyridinyl, thiazoylimidazolyl, thiopyrylyl, triazolyl, xanthyl and the ammonium salt thereof;
- ▪ a *cycloalkyl* radical is a monocyclic or fused or non-fused, bridged or non-bridged polycyclic hydrocarbon-based radical containing from 5 to 22 carbon atoms, such as cyclohexyl;
- ▪ a *cycloalkenyl* radical is a monocyclic or fused or non-fused, bridged or non-bridged polycyclic non-aromatic unsaturated hydrocarbon-based radical containing from 5 to 22 carbon atoms, such as cyclohexenyl;
- ▪ a *heterocycloalkyl* radical is a heterocyclic radical comprising at least one saturated ring;
- ▪ a *heterocyclic* radical is a 5- to 22-membered monocyclic or fused or non-fused polycyclic radical which may contain one or two unsaturations but is non-aromatic, comprising from 1 to 6 heteroatoms chosen from nitrogen, oxygen, sulfur and selenium atoms;
- ▪ an *alkyl* radical is a linear or branched $C_1$-$C_{20}$ and preferably $C_1$-$C_8$ hydrocarbon-based radical;
- ▪ the term *optionally substituted* attributed to the alkyl radical or a hydrocarbon-based aliphatic chain means that

said radical or said chain may be substituted with one or more radicals chosen from the following radicals: i) hydroxyl, ii) $C_1$-$C_4$ alkoxy, iii) acylamino, iv) amino optionally substituted with one or two identical or different $C_1$-$C_4$ alkyl radicals, said alkyl radicals possibly forming, with the nitrogen atom that bears them, a 5- to 7-membered heterocycle, optionally comprising another nitrogen or non-nitrogen heteroatom; v) or an aryl group such as phenyl;

- an *alkoxy* radical is an alkyl-oxy radical for which the alkyl radical is a linear or branched $C_1$-$C_{16}$ and preferentially $C_1$-$C_8$ hydrocarbon-based radical;
- when the alkoxy group is optionally substituted, this implies that the alkyl group is optionally substituted as defined hereinabove;
- the term *organic or mineral acid salt* more particularly means the salts chosen from a salt derived from i) hydrochloric acid HCl, ii) hydrobromic acid HBr, iii) sulfuric acid $H_2SO_4$, iv) alkylsulfonic acids: Alk-$S(O)_2OH$ such as methanesulfonic acid and ethanesulfonic acid; v) arylsulfonic acids: Ar-$S(O)_2OH$ such as benzenesulfonic acid and toluenesulfonic acid; vi) citric acid; vii) succinic acid; viii) tartaric acid; ix) lactic acid; x) alkoxysulfinic acids: Alk-O-S(O)OH such as methoxysulfinic acid and ethoxysulfinic acid; xi) aryloxysulfinic acids such as tolueneoxysulfinic acid and phenoxysulfinic acid; xii) phosphoric acid $H_3PO_4$; xiii) acetic acid $CH_3C(O)OH$; xiv) triflic acid $CF_3SO_3H$; and xv) tetrafluoroboric acid $HBF_4$;
- moreover, the addition salts that may be used in the context of the invention are especially chosen from addition salts with a cosmetically acceptable base such as basifying agents as defined below, for instance alkali metal hydroxides, such as sodium hydroxide or potassium hydroxide, aqueous ammonia, amines or alkanolamines;
- the expression *at least one* is equivalent to *one or more;* and
- the expression *inclusive* for a range of concentrations means that the limits of the range are included in the defined range.

*i) Nucleophilic alkoxysilane polymer or oligomer*

The process, the composition and the kit according to the invention use i) at least one nucleophilic alkoxysilane polymer or oligomer.

The term *nucleophilic alkoxysilane polymer or oligomer* means any alkoxysilane polymer or oligomer derived from the polymerization of a silane monomer which comprises a) at least one ($C_1$-$C_{10}$)alkoxy group and b) at least one "nucleophilic" group, i.e. an electron-donating group which are primary or secondary amino groups -N(H)$R_b$ with $R_b$ representing a hydrogen atom or an optionally substituted ($C_1$-$C_6$)alkyl group, a ($C_5$-$C_{10}$)cycloalkyl group, a 5- to 10-membered heterocycloalkyl group or a (hetero)aryl group.

The nucleophilic alkoxysilane polymer or oligomer of the invention is derived from a monomer of formula **(I)** below, and also the acid salts thereof, and the solvates thereof such as hydrates:

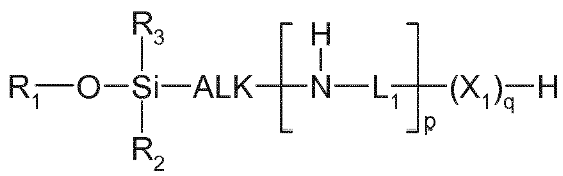

**(I)**

in which formula **(I):**

- **p** and **q,** which may be identical or different, are equal to 0 or 1, with the sum p + q being greater than or equal to 1, preferably p + q is equal to 1 or 2, in particular p is 0 and q is 1;
- $\mathbf{X_1}$ represents an amino group -N($R_a$)- with $R_a$ representing a hydrogen atom or a group chosen from ($C_1$-$C_8$)alkyl such as methyl, ($C_5$-$C_7$)cycloalkyl such as cyclohexyl, aryl such as phenyl, and aryl($C_1$-$C_4$)alkyl such as benzyl, in particular an amino group and preferably -N(H)-;
- **ALK** represents a linear or branched $C_1$-$C_{10}$, in particular linear, saturated divalent hydrocarbon-based chain, more particularly $-(CH_2)_x-$ with x representing an integer between 1 and 6 inclusive, preferably between 1 and 4 such as methylene, ethylene or propylene; more preferentially methylene or propylene, or even propylene;
- $\mathbf{R_1}$ represents a hydrogen atom or a ($C_1$-$C_6$)alkyl group such as methyl or ethyl;
- $\mathbf{R_2}$ and $\mathbf{R_3}$,which may be identical or different, preferably identical, represent a group chosen from hydroxyl, ($C_1$-$C_6$)alkyl and ($C_1$-$C_6$)alkoxy, in particular $C_1$-$C_4$ alkoxy such as ethoxy;
- $\mathbf{L_1}$ represents a linear or branched saturated $C_1$-$C_{20}$ divalent hydrocarbon-based chain; in particular linear, more particularly $-(CH_2)_x-$ with x as defined previously; in particular, $L_1$ is an ethylene or propylene group.

According to a particular embodiment of the invention, the nucleophilic alkoxysilane polymer(s) or oligomer(s) are derived from monomers of formula (I) as defined previously in which, taken together or separately:

- **p** is 0 and **q** is 1;
- $\mathbf{R_1}$ represents a ($C_1$-$C_4$)alkyl group such as ethyl;
- $\mathbf{R_2}$ and $\mathbf{R_3}$ are identical and represent a ($C_1$-$C_4$)alkoxy group such as ethoxy;
- **ALK** represents a methylene or propylene group, preferably propylene;
- $\mathbf{X_1}$ represents an amino group -N($R_a$)- as defined previously, in particular, $R_a$ representing a hydrogen atom or a ($C_1$-$C_4$)alkyl group; preferably, $R_a$ represents a hydrogen atom.

Advantageously, the nucleophilic alkoxysilane polymer or oligomer has a weight-average molecular weight ranging from 200 to 1 000 000 g/mol, preferably ranging from 300 to 500 000 g/mol.
Preferably, the nucleophilic alkoxysilane polymer or oligomer is water-soluble. The term "water-soluble polymer" means a polymer with a solubility in water, at 25°C, of at least 0.1 g/l.
The nucleophilic alkoxysilane polymer or oligomer may be present in the composition in which said polymer is contained in a content ranging from 0.001 % to 20% by weight, relative to the total weight of the composition in which said polymer is contained, preferably ranging from 0.001% to 10% by weight, preferentially ranging from 0.001 % to 5% by weight, and more preferentially ranging from 0.5% to 5% by weight.
Preferably, they are oligomers or polymers obtained by polymerization of APTES.

*ii) an activated (thio)ester*

The process, the composition and the kit according to the invention also use ii) at least one activated (thio)ester compound.
The term "activated (thio)ester compound" means an ester or thioester compound comprising an aliphatic chain of formula **R-C(Y)-Y'-A** with:

- **R** representing a linear or branched, saturated or unsaturated, preferably saturated, optionally substituted, preferably unsubstituted, hydrocarbon-based aliphatic chain, optionally interrupted with one or more heteroatoms such as oxygen, comprising from 5 to 30 carbon atoms;
- **Y** and **Y',** which may be identical or different, represent an oxygen or sulfur atom; in particular, Y' represents an oxygen atom; and
- **A** representing an activating group of the carbon atom C of the (thio)carbonyl group -C(Y)-, which is able to leave in the presence of a nucleophile, liberate A-Y'- and create a covalent bond between the carbon atom C of the -C(Y)-group and said nucleophile.

The group **A** is an optionally substituted heterocyclic group or an optionally substituted heteroaryl group linked to the rest of the molecule via a heteroatom such as nitrogen, or a (hetero)arylamino group.
According to a particular embodiment of the invention, the activated (thio)ester(s) are of formula **(II)** below, and also the solvates thereof such as hydrates:

**R'-C(Y)-OAi** **(II)**

in which formula **(II)**:

- **R'** represents a $C_5$-$C_{21}$, preferably $C_9$-$C_{17}$ and preferentially $C_{11}$-$C_{15}$ alkyl group;
- **Y** is as defined previously; preferably, Y represents an oxygen atom;
- $\mathbf{A_1}$ denotes a reactive group chosen from $\mathbf{A_{1a}}$, $\mathbf{A_{1b}}$ and $\mathbf{A_{1c}}$:

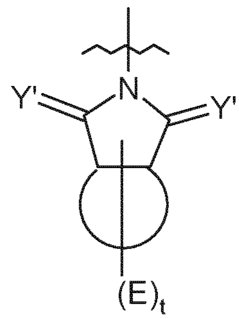

**$A_{1a}$**

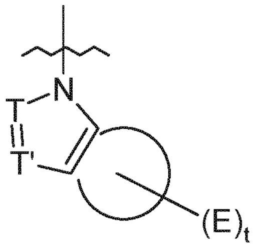

**$A_{1b}$**

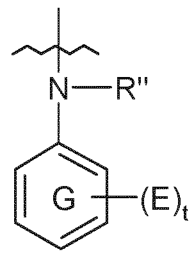

**$A_{1c}$**

with

- **Y',** which may be identical or different, preferably identical, representing an oxygen or sulfur atom, preferably oxygen;
- 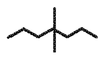

  representing the bond which links $A_{1a}$, $A_{1b}$ or $A_{1c}$ to the rest of the molecule;
- **T** and **T',** which may be identical or different, preferably identical, representing a methylene group $C(R^a)$ with $R^a$ representing a hydrogen atom or an optionally substituted ($C_1$-$C_6$)alkyl, or a nitrogen atom, in particular nitrogen, or alternatively T represents a group $C(R^a)$ such as CH and T' represents a nitrogen atom;
- **E,** which may be identical or different, when t is greater than or equal to 2, representing an atom or electron-withdrawing group such as halogen, nitro, nitroso, cyano, carboxyl, phosphate, polyhaloalkyl, such as trifluoromethyl, sulfoxy, $SO_3^-M^+$ with $M^+$ representing a hydrogen atom, or a cationic counterion such as alkali metal, alkaline-earth metal or ammonium, such as sodium or potassium; preferably, E represents a halogen atom such as fluorine or chlorine or a group $SO_3^-M^+$;
- **t** representing an integer between 0 and 5 inclusive;
- **G** representing an aryl group such as phenyl, or a heteroaryl group;
- 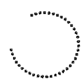 ,

  which may be present or absent, represents a (Cs-$C_{10}$)cycloalkyl, ($C_5$-$C_{10}$)cycloalkenyl, 5- to 10-membered heterocycloalkyl, 5- to 10-membered heterocycloalkenyl, aryl such as benzo, or heteroaryl monocycle or fused bicycle; preferably a fused bicyclic ($C_5$-$C_7$)cycloalkyl, bicyclic ($C_5$-$C_7$)cycloalkenyl or benzo group; and
- **R"** representing a hydrogen atom or an optionally substituted ($C_1$-$C_6$)alkyl group such as benzyl, or a (hetero)aryl group such as phenyl;

According to a particular embodiment of the invention, the polyalkoxylated activated (thio)ester(s) are of formula **(II)** as defined previously in which $A_1$ are chosen from **$A'_{1a}$, $A''_{1a}$, $A'''_{1a}$, $A''''_{1a}$, $A'_{1b}$, $A''_{1b}$** and **$A'_{1c}$:**

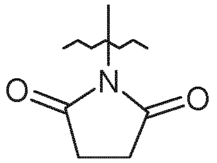

**A'$_{1a}$**

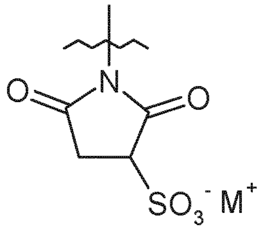

**A"$_{1a}$**

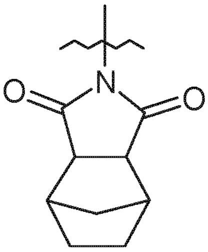

**A"'$_{1a}$**

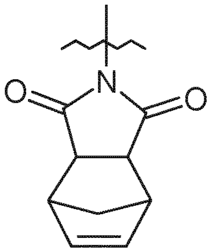

**A""$_{1a}$**

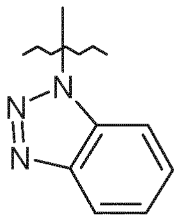

**A'$_{1b}$**

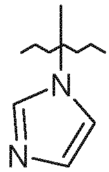

**A"$_{1b}$**

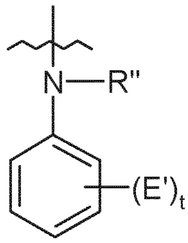

**A'$_{1c}$**

with:

- **$M^+$** representing a cationic counterion as defined previously, in particular representing an alkali metal such as sodium;
- **E',** which may be identical or different, when t is greater than or equal to 2, representing an electron-withdrawing group such as a halogen atom, chosen in particular from fluorine and chlorine;
- **t** is an integer between 0 and 5 inclusive;
- **R"** being as defined previously, in particular representing a hydrogen atom or a ($C_1$-$C_6$)alkyl group, preferably a hydrogen atom.

Preferably, **$A_1$** represents a group **A'$_{1a}$,A"$_{1a}$**, or **A"$_{1b}$**, preferably **A'$_{1a}$**.

These activated (thio)ester compounds are known from the literature and to those skilled in the art.

More particularly, **$A_1$** represents a group **A'$_{1a}$** or **A"$_{1a}$**, preferably **A'$_{1a}$** and **R'** represents a $C_9$-$C_{17}$ and preferentially $C_{11}$-$C_{15}$ alkyl group.

Among the reactive groups, the activated (thio)ester compound may be:
the esters of lauric, palmitic, pentanoic, hexanoic, 2-ethylhexanoic, octanoic, nonanoic, decanoic, dodecanoic, hexadecanoic, eicosanoic, hexacosanoic, octadecenoic, undecenoic, eicosatetraenoic or octadecatrienoic acid and of *N*-hydroxysuccinimide,

The activated (thio)ester compound is preferably chosen from: the ester of lauric acid and of *N*-hydroxysuccinimide, the ester of palmitic acid and of *N*-hydroxysuccinimide. Advantageously, the activated (thio)ester compound is the ester of palmitic acid and of *N*-hydroxysuccinimide. The activated (thio)ester compound may be present in the composition in which said activated (thio)ester compound is contained in a content ranging from 0.1% to 5% by weight, relative to the total weight of the composition in which said activated (thio)ester compound is contained, preferably ranging from 0.5% to 4% by weight, and preferentially ranging from 1% to 4% by weight.

*Composition (C) or compositions (A) and (B)*

Composition (C) or compositions (A) and (B) used according to the invention contain a physiologically acceptable medium, i.e. a medium that is compatible with human keratin materials such as the skin (of the body, face, eye contour area or the scalp), the hair, the eyelashes, the eyebrows, bodily hair, the nails or the lips.

Advantageously, the cosmetic composition (A) according to the invention comprises a physiologically acceptable aqueous medium. It may be constituted, for example, of water or of a mixture of water and of at least one cosmetically acceptable organic solvent. Examples of organic solvents that may be mentioned include $C_2$-$C_4$ lower alcohols, such as ethanol and isopropanol; polyols, especially those containing from 2 to 6 carbon atoms, for instance glycerol, propylene glycol, butylene glycol, pentylene glycol, hexylene glycol, dipropylene glycol or diethylene glycol; polyol ethers, for

instance 2-butoxyethanol, propylene glycol monomethyl ether, diethylene glycol monomethyl ether or monoethyl ether, and short esters such as ethyl acetate or butyl acetate; and mixtures thereof. Preferably, the cosmetic composition comprises from 50% to 99.5% by weight of water relative to the weight of the composition.

Advantageously, the cosmetic composition (B) used according to the invention comprises a physiologically acceptable non-aqueous medium. It may be constituted, for example, by one or more cosmetically acceptable organic solvents, such as those described previously, or alternatively one or more common cosmetic oils.

Advantageously, the cosmetic composition (C) comprises a physiologically acceptable aqueous medium. It may be constituted, for example, by water or by a mixture of water and of at least one cosmetically acceptable organic solvent as described previously, or alternatively one or more common cosmetic oils. Preferably, the cosmetic composition (C) comprises from 40% to 99.5% by weight of water relative to the weight of the composition.

Composition (C) or compositions (A) and (B) used according to the invention may also contain one or more cosmetic additives chosen from nonionic, anionic, cationic and amphoteric surfactants, vitamins and provitamins, including panthenol, sunscreens, fillers, colorants, nacreous agents, opacifiers, sequestrants, film-forming polymers, plasticizers, thickeners, aliphatic or silicone oils, antioxidants, antifoams, moisturizers, emollients, penetrants, fragrances and preserving agents.

Composition (C) or compositions (A) and (B) used according to the invention may be in any galenical form conventionally used for application to the hair and especially in the form of aqueous solutions, aqueous-alcoholic solutions, oil-in-water (O/W), water-in-oil (W/O) or multiple (triple: W/O/W or O/W/O) emulsions, aqueous gels or aqueous-alcoholic gels. These compositions are prepared according to the usual methods. Preferably, the composition is in the form of an aqueous or aqueous-alcoholic solution or gel.

*The process for treating keratin fibres*

The process according to the invention is performed on keratin fibres, especially human keratin fibres such as the hair. Said fibres may be wet or dry. Preferentially, the process is performed on dry keratin fibres, especially hair.

According to a particular embodiment of the invention, ingredients i) and ii) as defined previously are together in the same cosmetic composition (C) that is then applied to the keratin fibres. In this case, composition (C) may originate from the extemporaneous mixing of compositions (A) and (B). According to one embodiment, compositions (A) and (B) are applied together to the keratin fibres, compositions (A) and (B) may be mixed together before application to give a composition (C), said composition (C) then being applied to the keratin fibres.

According to a particularly advantageous embodiment, compositions (A) and (B) are applied to the keratin fibres separately.

According to one embodiment of the process of the invention, composition (B) is applied first to the keratin fibres, and composition (A) is subsequently applied to the keratin fibres.

According to a preferred embodiment of the process of the invention, composition (A) is applied to the keratin fibres and composition (B) is subsequently applied to the keratin fibres.

Preferably, when the process for treating keratin fibres of the invention is performed in several steps, between the step of applying composition (A) and composition (B), or between the step of applying composition (B) and composition (A), no intermediate rinsing with water is performed.

According to a particularly preferred embodiment of the process of the invention, composition (A) is applied to the keratin fibres, then keratin fibres are dried, and composition (B) is subsequently applied to the keratin fibres.

According to another embodiment of the process of the invention, composition (B) is applied first to the keratin fibres, then keratin fibres are dried and composition (A) is subsequently applied to the keratin fibres.

The drying step is preferably complete. The latter can be performed at room temperature or by heat treatment, preferably at room temperature. The heat treatment is conducted at a temperature between 30 and 250 °C until completely dry. In practice, this can be conducted using a hair helmet, hair dryer, a curl or flat iron, an infrared rays dispenser or other heating devices and under plastic film, preferably with a domestic hairdryer in particular at a temperature between 50 °C and 100 °C.

After application to the keratin fibres of the cosmetic composition (A), (B) or (C), said applied composition may be left to stand on the fibres for a time ranging from 1 to 60 minutes, preferably ranging from 2 to 50 minutes, preferentially ranging from 5 to 30 minutes. The leave-on time may take place at a temperature ranging from 15°C to 45°C, preferably at room temperature (25°C).

The cosmetic composition(s) described previously are advantageously applied to the keratin fibres in an amount ranging from 0.1 to 10 grams and preferably from 0.2 to 5 grams of composition per gram of keratin fibres.

After application of the cosmetic compositions to the keratin fibres, the latter may be drained dry to remove the excess composition or washed with water.

After the treatment, the keratin fibres may be optionally rinsed with water or washed with a shampoo. The keratin fibres are then optionally dried with a hairdryer or a hood or in the open air.

The treatment process according to the invention is preferably performed on keratin fibres, especially hair, which are sensitized, such as artificially dyed fibres (keratin fibres dyed following a direct dyeing process or via an oxidation dyeing process), bleached, relaxed or permanent-waved fibres.

The treatment process according to the invention may be performed before, during and/or after an additional process of cosmetic treatment of the keratin fibres, such as a process for temporarily shaping (shaping with curlers, a crimping iron or a straightening iron) or a process for durably shaping (permanent-waving or relaxing) the keratin fibres.

The treatment process may be performed as a pre-treatment to a dyeing or relaxing process and/or a permanent-waving process so as to cosmetically protect the keratin fibres against these treatments. In other words, this process is performed to preserve the cosmetic properties of the keratin fibres before a cosmetic treatment process as described previously.

Preferentially, the treatment process is performed as a post-treatment to a bleaching, artificial dyeing or relaxing process and/or a permanent-waving process so as to repair said fibres.

The process according to the invention is preferably performed on the hair.

The examples that follow are given as illustrations of the present invention.

The amounts indicated in the examples are expressed as weight percentages.

## EXAMPLES

### Process for treating keratin fibres

Process 1 in 2 steps:

*1) Treatment of the keratin fibre lock with a solution A of nucleophilic alkoxysilane polymer:*

The lock was moistened, and 1.5 g of an aqueous solution of (3-aminopropyl)triethoxysilane (APTES) at 10% by weight/g of hair were then deposited (the aqueous composition with APTES was applied by pipette and then by brush onto the lock, in a relatively wide plate, so as to spread out the lock as much as possible).

The lock was then placed in an oven at 40°C for 30 minutes covered with aluminium foil to prevent evaporation, and the lock was then turned over after 15 minutes without intermediate rinsing.

*2) Subsequent treatment of the keratin fibre lock with a solution B of activated (thio)ester compound*

A solution of "Palmitic NHS", ester of palmitic acid and of *N*-hydroxysuccinimide, at 3% by weight in butyl acetate/ethanol medium (50/50 v/v) was prepared, and 1.5 g of this solution/g of hair were then deposited. The application method is identical to that of the preceding step.

The lock was then placed in an oven at 40°C for 60 minutes covered with aluminium foil to prevent evaporation, and the lock was then turned over after 30 minutes. The lock was divided into two, one part was rinsed and the other was not.

Process 2 in 2 steps:

*1) Treatment of the keratin fibre lock with a solution B of activated (thio)ester compound*

The lock was moistened, and 1.5 g of a solution of "Palmitic NHS" at 3% by weight in butyl acetate/ethanol medium (50/50 v/v)/g of hair were then applied. The application method and the conditions are identical to those of step 1) of the preceding process 1.

*2) Subsequent treatment of the keratin fibre lock with a solution A of nucleophilic alkoxysilane polymer:*

1.5 g of an aqueous solution of APTES at 10% by weight/g of hair were applied under the same experimental conditions as in step 2) of the preceding process 1.

Process 3 in 1 step:

Preparation of composition C:

A solution of APTES at 10% by weight in a butyl acetate/ethanol mixture (50/50 v/v) is prepared. Another solution comprising Palmitic NHS at 3% by weight in a butyl acetate/ethanol mixture (50/50 v/v) is prepared. The two

solutions are then mixed together in a 50/50 v/v amount, to give composition C.

3 g of composition C are then applied to 1 g of a lock of hair. The application method and the experimental conditions are identical to those of step 2) of the preceding processes 1 and 2.

Process 4 in 3 steps:

*1) Treatment of the keratin fibre lock with a solution A of nucleophilic alkoxysilane polymer:*

The lock was moistened, and 1.5 g of an aqueous solution of (3-aminopropyl)triethoxysilane (APTES) at 10% by weight/g of hair were then deposited. The application method and the conditions are identical to those of step 1) of the preceding process 1.

*2) Subsequent drying of the keratin fibre lock at room temperature (25°C)*

*3) Subsequent treatment of the keratin fibre lock with a solution B of activated (thio)ester compound*

A solution of "Palmitic NHS", ester of palmitic acid and of *N*-hydroxysuccinimide, at 3% by weight in butyl acetate/ethanol medium (50/50 v/v) was prepared, and 1.5 g of this solution/g of hair were then deposited. The application method is identical to that of the preceding step.

The lock was then placed in an oven at 40 °C for 60 minutes covered with aluminium foil to prevent evaporation, and the lock was then turned over after 30 minutes. The lock was then rinsed once with a shampoo.

**Evaluations**

The various conditions that were used are as follows:
Four controls/placebos:

- $H_2O$ control corresponding to the untreated hair
- Mixture of butyl acetate/ethanol (50/50 v/v) corresponding to the palmitic-NHS solvent for examination of the reactivity of the solvent alone
- APTES (10% by weight in water $H_2O$) for examination of the reactivity of "APTES" alone
- palmitic-NHS (noted -NHS) (3% by weight in a butyl acetate/ethanol mixture (50/50 v/v)) for examination of the reactivity of NHS alone

Four treatment conditions were implemented:

- Condition in 2 stages: APTES (10%) followed by palmitic-NHS (3%) corresponding to process 1
- Condition in 2 stages: palmitic-NHS (3%) then APTES (10%) corresponding to process 2
- Condition in 1 stage: aqueous solution of APTES (10%) and palmitic-NHS (3%) corresponding to process 3
- Condition in 3 stage: aqueous solution of APTES (10%) followed by a drying step at room temperature and followed by palmitic-NHS (3%) corresponding to process 4

The locks were treated with the compositions prepared according to the protocol and the process described above.

| Locks | Treatment | Invention/C omparative |
|---|---|---|
| Lock 1 | $H_2O$ - Control | Comparative |
| Lock 2 | Butyl acetate/ethanol (50/50 v/v) | Comparative |
| Lock 3 | Palmitic NHS (3% by mass in a 50/50 v/v butyl acetate/ethanol mixture) | Comparative |

(continued)

| Locks | Treatment | Invention/C omparative |
|---|---|---|
| Lock 4 | "APTES" water into which 10% by weight of APTES was introduced | Comparative |
| Lock 5 | Process 1 - APTES followed by palmitic NHS | Invention |
| Lock 6 | Process 2 - palmitic NHS followed by APTES | Invention |
| Lock 7 | Process 3 - APTES and palmitic NHS together | Invention |
| Lock 8 | Process 4 - APTES followed by a drying step at room temperature and followed by palmitic-NHS | Invention |

Wetting measurements: surface hvdrophobicitv/hvdrophilicitv

For each lock of treated hair, the wettability of the hair was measured (measurements described especially in the book The Science of Hair Care by C. Bouillon and J. Wilkinson - 2nd edition 2005 - Chapter 12: Evaluation of product efficacy - page 407).

The measurement of the wettability of a hair consists in immersing a piece of hair in a crystallizing dish of ultra-pure water and in measuring the force generated by the displacement of the hair fibre during its immersion (wetting force) and during its withdrawal from the water. This force varies as a function of the affinity of the hair for the liquid and makes it possible to assess the surface state of the fibre.

A K14 tensiometer from the company Krüss was used under the following operating conditions:

- Approach speed: 4 mm/minute.
- Measuring speed: 2 mm/minute.
- Balance sensitivity: 10 mg
- Waiting time after immersion: 2 seconds
- Immersion depth: 2 mm

The hairs were raised on a holder still oriented in the same direction, i.e. end downwards, root upwards.

For each treatment, 20 samples were measured under the same conditions.

The hairs were left to regulate overnight in a glove box at 25°C and 45% hygrometry before measuring them.

Data processing

The wetting force (F in newtons) was determined according to the following formula:

$$F = L \times \sigma \times \cos\theta$$

L being the perimeter of the hair (in metres)
$\theta$: contact angle
$\Sigma$: surface tension of pure water = 72.75 mN/m at atmospheric pressure

The wetting force expressed in mN was transformed into mg, and then normalized relative to the mean perimeter of the fibres.

From the 20 measurements for each lock, the mean value and its confidence interval were calculated. The lower the wetting force, the more hydrophobic the treated hair (less frizziness, better manageability, easier to style, volume control).

Friction measurements: slipperiness

The slipperiness of a treatment according to the invention is measured using a friction banc compared with a $H_2O$ control (corresponding to the untreated hair) and an APTES control (10% by weight in water $H_2O$).

Conditions for measuring 2 hair:

- Base weight: P= 11g

- Measurement length : 3mm
- Friction Speed :1mm/min
- Frequency : 20 Hz
- RH : 50%

Total number of measurements 16 hairs / condition and treatment

The frictional force between the blade and the moving hairs is then measured. More friction force between two surfaces, the lower the slipperiness of the treatment is important. The friction force is expressed in Newtons (N), and friction coefficient m is calculated according to the following formula: *Amonton's law:*

$$F = \mu \times P$$

**Results:**

It was seen that process 1 for treating keratin fibres made it possible to obtain smooth-feeling hair combining a body effect and coating. In addition, it was observed that this sensation persists even after several shampoo washes.

| Locks | No shampoo washes | Rinsing + 1 shampoo wash | Rinsing + 5 shampoo washes |
|---|---|---|---|
| | F (mg) | F (mg) | F (mg) |
| 1 (control) | 7.53 $_{6}$ 0.99 | 9.84 $_{6}$ 0.52 | 6.86 $_{6}$ 0.52 |
| 2 (comparative) | 8.53 $_{6}$ 0.63 | 8.92 $_{6}$ 0.34 | 7.50 $_{6}$ 0.45 |
| 3 (comparative) | 8.38 $_{6}$ 0.63 | 6.94 $_{6}$ 0.44 | 4.15 $_{6}$ 0.36 |
| 4 (comparative) | -2.11 $_{6}$ 0.98 | -0.61 $_{6}$ 0.83 | -5.35 $_{6}$ 0.98 |
| 5 (invention) | -11.64 $_{6}$ 0.73 | -6.39 $_{6}$ 0.59 | -6.46 $_{6}$ 0.21 |
| 6 (invention) | -9.84 $_{6}$ 0.95 | -7.82 $_{6}$ 0.47 | -2.18 $_{6}$ 0.32 |
| 7 (invention) | -4.17 $_{6}$ 1.16 | -9.29 $_{6}$ 0.37 | -4.96 $_{6}$ 0.31 |

It was seen that the process according to the invention made it possible significantly to afford long-lasting hydrophobic nature to the surface of the keratin fibres, and also a good feel.

It was seen that the process 4 for treating keratinous fibres, according to the invention, provides improved slipperiness, and therefore gives the hair a smooth-feeling and makes it easier to detangle the hair.

| Locks | Frictional force Direction: from root to tip | Frictional force Direction : from tip to root |
|---|---|---|
| Lock 1 - comparative | 0.208 | 0.227 |
| Lock 4 - comparative | 0.194 | 0.200 |
| Lock 8 - invention | 0.173 | 0.196 |

The slipperiness of the treatment, according to the invention, is very attractive, since it has a friction force less than the controls, regardless of the measuring direction.

## Claims

**1.** Process for treating keratin fibres, especially human keratin fibres such as the hair, comprising:

i) application to the keratin fibres of a cosmetic composition (A) comprising at least one nucleophilic alkoxysilane polymer or oligomer, in which the nucleophilic alkoxysilane polymer(s) or oligomer is derived from a monomer of formula **(I)** below, and also the acid salts thereof, and the solvates thereof such as hydrates:

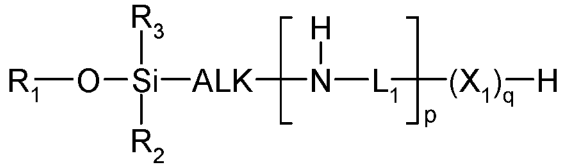

**(I)**

in which formula **(I):**

- **p** and **q,** which may be identical or different, are equal to 0 or 1, with the sum p + q being greater than or equal to 1, preferably p + q is equal to 1 or 2, in particular p is 0 and q is 1;
- $\mathbf{X_1}$ represents an amino group $-N(R_a)-$ with $R_a$ representing a hydrogen atom or a group chosen from $(C_1-C_8)$alkyl such as methyl, $(C_5-C_7)$cycloalkyl such as cyclohexyl, aryl such as phenyl, and aryl$(C_1-C_4)$alkyl such as benzyl, preferably -N(H)-;
- **ALK** represents a linear or branched $C_1-C_{10}$, in particular linear, saturated divalent hydrocarbon-based chain, more particularly $-(CH_2)_x-$ with x representing an integer between 1 and 6 inclusive, preferably between 1 and 4 such as methylene, ethylene or propylene; more preferentially methylene or propylene;
- $\mathbf{R_1}$ represents a hydrogen atom or a $(C_{1-}C_6)$alkyl group such as methyl or ethyl;
- $\mathbf{R_2}$ and $\mathbf{R_3}$, which may be identical or different, preferably identical, represent a group chosen from hydroxyl, $(C_{1-}C_6)$alkyl and $(C_{1-}C_6)$alkoxy, in particular $C_1-C_4$ alkoxy such as ethoxy;
- $\mathbf{L_1}$ represents a linear or branched saturated $C_{1-}C_{20}$ divalent hydrocarbon-based chain; in particular linear, more particularly $-(CH_2)_x-$ with x as defined previously; in particular, $L_1$ represents an ethylene or propylene group; and

ii) application of a cosmetic composition (B) comprising at least one activated (thio)ester compound of formula **R-C(Y)-Y'-A** with:

- **R** representing a linear or branched, saturated or unsaturated, preferably saturated, optionally substituted, preferably unsubstituted, hydrocarbon-based aliphatic chain, optionally interrupted with one or more heteroatoms such as oxygen, comprising from 5 to 30 carbon atoms;
- **Y** and **Y'**, which may be identical or different, represent an oxygen or sulfur atom; in particular, Y' represents an oxygen atom; and
- **A** representing an activating group of the carbon atom C of the (thio)carbonyl group -C(Y)-, which is able to leave in the presence of a nucleophile, liberate $A-Y'^-$ and create a covalent bond between the carbon atom C of the -C(Y)-group and said nucleophile.

it being understood that compositions (A) and (B) may be applied to the keratin fibres together or separately, preferably separately;
in which the activated (thio)ester compound(s) are such that the group **A** is:

- an optionally substituted heterocyclic group linked to the rest of the molecule via a heteroatom such as nitrogen;
- an optionally substituted heteroaryl group linked to the rest of the molecule via a heteroatom such as nitrogen; or
- a (hetero)arylamino group.

**2.** Process according to the preceding claim, in which the nucleophilic alkoxysilane polymer(s) or oligomer comprise one or more nucleophilic groups chosen from primary or secondary amino groups $-N(H)R_b$ with $R_b$ representing a hydrogen atom or an optionally substituted $(C_{1-}C_6)$alkyl group, a $(C_5-C_{10})$cycloalkyl group, a 5- to 10-membered heterocycloalkyl group or a (hetero)aryl group, preferably $R_b$ representing a hydrogen atom.

**3.** Process according to any one of the preceding claims, in which the nucleophilic alkoxysilane polymer(s) or oligomer derived from a monomer of formula (I) are, and also the acid salts thereof, and the solvates thereof such as hydrates, taken together or separately:

- **p** is 0 and **q** is 1;
- $\mathbf{R_1}$ represents a $(C_1-C_4)$alkyl group such as ethyl;
- $\mathbf{R_2}$ and $\mathbf{R_3}$ are identical and represent a $(C_1-C_4)$alkoxy group such as ethoxy;

- **ALK** represents a methylene or propylene group;
- **$X_1$** represents an amino group -N($R_a$)- as defined previously, in particular, $R_a$ representing a hydrogen atom or a ($C_1$-$C_4$)alkyl group; preferably, $R_a$ represents a hydrogen atom.

**4.** Process according to any one of the preceding claims, in which the activated (thio)ester compound(s) are of formula **(II)** below, and also the solvates thereof such as hydrates:

**R'-C(Y)-OA$_1$** **(II)**

in which formula **(II):**

- **R'** represents a $C_5$-$C_{21}$, preferably $C_9$-$C_{17}$ and preferentially $C_{11}$-$C_{15}$ alkyl group;
- **Y** is as defined previously; preferably, Y represents an oxygen atom;
- **$A_1$** denotes a reactive group chosen from **$A_{1a}$**, **$A_{1b}$** and **$A_{1c}$**:

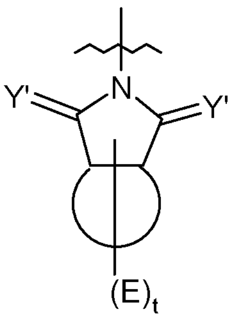

**$A_{1a}$**

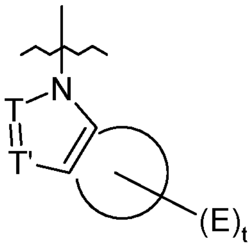

**$A_{1b}$**

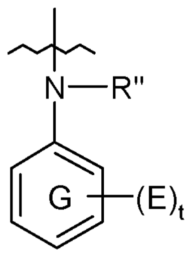

**$A_{1c}$**

with

- **Y'**, which may be identical or different, preferably identical, representing an oxygen or sulfur atom, preferably oxygen;
- 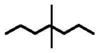 representing the bond which links $A_{1a}$, $A_{1b}$ or $A_{1c}$ to the rest of the molecule;
- **T** and **T'**, which may be identical or different, preferably identical, representing a methylene group C($R^a$) with $R^a$ representing a hydrogen atom or an optionally substituted ($C_1$-$C_6$)alkyl, or a nitrogen atom, in particular nitrogen, or alternatively T represents a group C($R^a$) such as CH and T' represents a nitrogen atom;
- **E**, which may be identical or different, when t is greater than or equal to 2, representing an atom or electron-withdrawing group such as halogen, nitro, nitroso, cyano, carboxyl, phosphate, polyhaloalkyl, such as trifluoromethyl, sulfoxy, $SO_3^-M^+$ with $M^+$ representing a hydrogen atom, or a cationic counterion such as alkali metal, alkaline-earth metal or ammonium, such as sodium or potassium; preferably, E represents a halogen atom such as fluorine or chlorine or a group $SO_3^-M^+$;
- **t** representing an integer between 0 and 5 inclusive;
- **G** representing an aryl group such as phenyl, or a heteroaryl group;
- 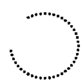,

which may be present or absent, represents a ($C_5$-$C_{10}$)cycloalkyl, ($C_5$-$C_{10}$)cycloalkenyl, 5- to 10-membered heterocycloalkyl, 5- to 10-membered heterocycloalkenyl, aryl such as benzo, or heteroaryl monocycle or fused bicycle; preferably a fused bicyclic ($C_5$-$C_7$)cycloalkyl, bicyclic ($C_5$-$C_7$)cycloalkenyl or benzo group; and
- **R"** representing a hydrogen atom or an optionally substituted ($C_1$-$C_6$)alkyl group such as benzyl, or a (hetero)aryl group such as phenyl.

**5.** Process according to the preceding claim, in which the activated (thio)ester compound(s) of formula **(II)**, and also the solvates thereof such as hydrates, are such that $\mathbf{A_1}$ is chosen from $\mathbf{A'_{1a}}$, $\mathbf{A''_{1a}}$, $\mathbf{A'''_{1a}}$, $\mathbf{A''''_{1a}}$, $\mathbf{A'_{1b}}$, $\mathbf{A''_{1b}}$ and $\mathbf{A'_{1c}}$:

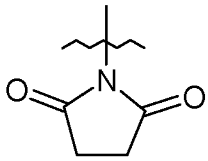

$\mathbf{A'_{1a}}$

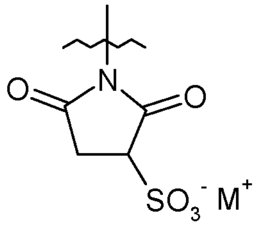

$\mathbf{A''_{1a}}$

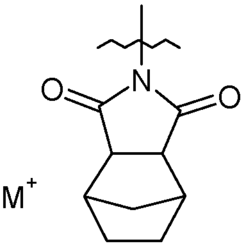

$\mathbf{A'''_{1a}}$

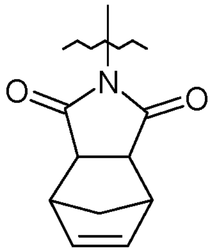

$\mathbf{A''''_{1a}}$

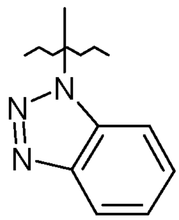

$\mathbf{A'_{1b}}$

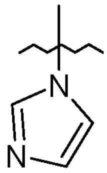

$\mathbf{A''_{1b}}$

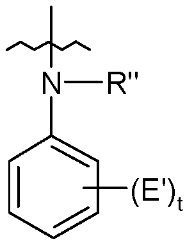

$\mathbf{A'_{1c}}$

with:

- $\mathbf{M^+}$ representing a cationic counterion as defined previously, in particular representing an alkali metal such as sodium;
- **E'**, which may be identical or different, when t is greater than or equal to 2, representing an electron-withdrawing group such as a halogen atom, chosen in particular from fluorine and chlorine;
- **t** is an integer between 0 and 5 inclusive;
- **R"** being as defined previously, in particular representing a hydrogen atom or a $(C_1\text{-}C_6)$alkyl group, preferably a hydrogen atom;

preferably, $\mathbf{A_1}$ represents a group $\mathbf{A'_{1a}}$, $\mathbf{A''_{1a}}$, or $\mathbf{A''_{1b}}$, preferably $\mathbf{A'_{1a}}$.

**6.** Process according to either of Claims 4 and 5, in which the activated (thio)ester compound(s) of formula **(II)** are such that $\mathbf{A_1}$ represents a group $\mathbf{A'_{1a}}$ or $\mathbf{A''_{1a}}$, preferably $\mathbf{A'_{1a}}$ and **R'** represents a $C_9$-$C_{17}$ and preferentially $C_{11}$-$C_{15}$ alkyl group.

**7.** Process according to any one of the preceding claims, in which the activated (thio)ester compound(s) are chosen from: the esters of lauric, palmitic, pentanoic, hexanoic, 2-ethylhexanoic, octanoic, nonanoic, decanoic, dodecanoic, hexadecanoic, eicosanoic, hexacosanoic, octadecenoic, undecenoic, eicosatetraenoic or octadecatrienoic acid and of *N*-hydroxysuccinimide.

**8.** Process according to any one of the preceding claims, in which the activated (thio)ester compound(s) are present in a content ranging from 0.1% to 5% by weight, relative to the total weight of the composition in which said activated (thio)ester compounds are contained, preferably ranging from 0.5% to 4% by weight, and preferentially ranging from 1% to 4% by weight.

**9.** Process according to any one of the preceding claims, in which compositions (A) and (B) are applied together to the keratin fibres, compositions (A) and (B) may be mixed together before application to give a composition (C), said composition (C) then being applied to the keratin fibres.

**10.** Process according to any one of Claims 1 to 8, in which composition (B) is applied first, and composition (A) is

subsequently applied; preferably, between the step of application of composition (B) and composition (A), no intermediate rinsing with water is performed.

**11.** Process according to any one of Claims 1 to 8, in which composition (A) is applied first, and composition (B) is subsequently applied; preferably, between the step of application of composition (A) and composition (B), no intermediate rinsing with water is performed.

**12.** Process according to any one of Claims 10 or 11 wherein between the step of applying the composition of step (B) and the composition (A) or between the application step of the composition (A) and the composition (B) it is carried out a step for drying the keratin fibers ; the drying step is preferably complete, it can be performed at room temperature or by heat treatment, preferably at room temperature ; said heat treatment is performed at a temperature between 30 and 250 °C especially with a hair helmet, hair dryer, a curl iron or iron flat, an infrared dispenser or other heating devices, and in plastic film, preferably with a domestic hairdryer especially at a temperature of 50 °C and 100 °C.

**13.** Process according to any one of the preceding claims, in which, after the application to the keratin fibres of the cosmetic composition (A), (B) or (C), said applied composition is left to stand on the fibres for a time ranging from 1 to 60 minutes, preferably ranging from 2 to 50 minutes, preferentially ranging from 5 to 30 minutes; the leave-on time may take place at a temperature ranging from 15°C to 45°C, preferably at room temperature (25°C); preferably, after the treatment, the keratin fibres are rinsed with water or washed with a shampoo, the keratin fibres are then optionally dried with a hairdryer or a hood or in the open air; in particular, the process may be performed before, during and/or after an additional process of cosmetic treatment of the keratin fibres, such as a temporary shaping process, especially shaping with curlers, a crimping iron or a straightening iron, or a long-lasting shaping process, especially permanent-waving or relaxing of the keratin fibres.

**14.** Cosmetic composition comprising:

i) at least one nucleophilic alkoxysilane polymer as defined in any one of Claims 1 to 3; and
ii) at least one activated (thio)ester compound of formula **R-C(Y)-Y'-A** with:

- **R** representing a linear or branched, saturated or unsaturated, preferably saturated, unsubstituted, hydrocarbon-based aliphatic chain, optionally interrupted with one or more heteroatoms such as oxygen, comprising from 5 to 30 carbon atoms;
- **Y** and **Y',** which may be identical or different, represent an oxygen or sulfur atom; in particular, Y' represents an oxygen atom; and
- **A** representing an activating group of the carbon atom C of the (thio)carbonyl group -C(Y)-, which is able to leave in the presence of a nucleophile, liberate $A\text{-}Y'^{-}$ and create a covalent bond between the carbon atom C of the -C(Y)-group and said nucleophile;

in which the activated (thio)ester compound(s) are such that the group **A** is:

- an optionally substituted heterocyclic group linked to the rest of the molecule via a heteroatom such as nitrogen;
- an optionally substituted heteroaryl group linked to the rest of the molecule via a heteroatom such as nitrogen; or
- a (hetero)arylamino group.

**15.** Multi-compartment device or kit, comprising, in a first compartment:

i) at least one nucleophilic alkoxysilane polymer as defined in any one of Claims 1 to 3; and
in a second compartment:
ii) at least one activated (thio)ester compound of formula **R-C(Y)-Y'-A** with:

- **R** representing a linear or branched, saturated or unsaturated, preferably saturated, unsubstituted, hydrocarbon-based aliphatic chain, optionally interrupted with one or more heteroatoms such as oxygen, comprising from 5 to 30 carbon atoms;
- **Y** and **Y',** which may be identical or different, represent an oxygen or sulfur atom; in particular, Y' represents an oxygen atom; and
- **A** representing an activating group of the carbon atom C of the (thio)carbonyl group -C(Y)-, which is able

to leave in the presence of a nucleophile, liberate A-Y'⁻ and create a covalent bond between the carbon atom C of the -C(Y)-group and said nucleophile;

in which the activated (thio)ester compound(s) are such that the group **A** is:

- an optionally substituted heterocyclic group linked to the rest of the molecule via a heteroatom such as nitrogen;
- an optionally substituted heteroaryl group linked to the rest of the molecule via a heteroatom such as nitrogen; or
- a (hetero)arylamino group;

ingredients i) and ii) each being packaged in a separate packaging assembly.

## Patentansprüche

**1.** Verfahren zur Behandlung von Keratinfasern, insbesondere menschlichen Keratinfasern wie dem Haar, umfassend:

i) das Aufbringen einer kosmetischen Zusammensetzung (A), die mindestens ein nucleophiles Alkoxysilan-Polymer oder -Oligomer umfasst, auf die Keratinfasern, wobei sich das nucleophile Alkoxysilan-Polymer oder -Oligomer bzw. die nucleophilen Alkoxysilan-Polymere oder -Oligomere von einem Monomer der nachstehenden Formel **(I)** sowie den Säuresalzen davon und den Solvaten davon wie Hydraten ableitet bzw. ableiten:

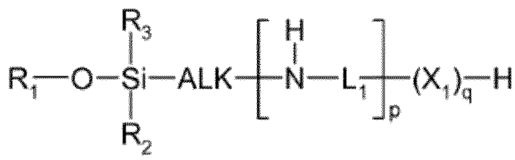

**(I)**

wobei in Formel **(I)**:

- **p** und **q,** die gleich oder verschieden sein können, gleich 0 oder 1 sind, wobei die Summe p + q größer oder gleich 1 ist, vorzugsweise p + q gleich 1 oder 2 ist, insbesondere p für 0 steht und q für 1 steht;
- **$X_1$** für eine Aminogruppe -N($R_a$)-, wobei $R_a$ für ein Wasserstoffatom oder eine aus ($C_1$-$C_8$)Alkyl wie Methyl, ($C_5$-$C_7$)Cycloalkyl wie Cyclohexyl, Aryl wie Phenyl und Aryl($C_1$-$C_4$)alkyl wie Benzyl, ausgewählt ist, vorzugsweise -N(H)-, steht;
- **ALK** für eine lineare oder verzweigte, insbesondere lineare, gesättigte zweiwertige $C_1$-$C_{10}$-Kette auf Kohlenwasserstoffbasis, spezieller $-(CH_2)_x$, wobei x für eine ganze Zahl zwischen 1 und 6 inklusive, vorzugsweise zwischen 1 und 4, steht, wie Methylen, Ethylen oder Propylen, weiter bevorzugt Methylen oder Propylen, steht;
- **$R_1$** für ein Wasserstoffatom oder eine ($C_1$-$C_6$)-Alkylgruppe wie Methyl oder Ethyl steht;
- **$R_2$** und **$R_3$**, die gleich oder verschieden sein können und vorzugsweise gleich sind, für eine aus Hydroxyl, ($C_1$-$C_6$)Alkyl und ($C_1$-$C_6$) Alkoxy ausgewählte Gruppe, insbesondere $C_1$-$C_4$-Alkoxy wie Ethoxy, stehen;
- **$L_1$** für eine lineare oder verzweigte gesättigte zweiwertige $C_1$-$C_{20}$-Kette auf Kohlenwasserstoffbasis, die insbesondere linear ist, spezieller $-(CH_2)_x$, wobei x wie oben definiert ist, steht; $L_1$ insbesondere für eine Ethylen- oder Propylengruppe steht; und

ii) das Aufbringen einer kosmetischen Zusammensetzung (B), die mindestens eine aktivierte (Thio)esterverbindung der Formel **R-C(Y)-Y'-A** umfasst, wobei:

- **R** für eine lineare oder verzweigte, gesättigte oder ungesättigte, vorzugsweise gesättigte, gegebenenfalls substituierte, vorzugsweise unsubstituierte, aliphatische Kette auf Kohlenwasserstoffbasis, die gegebenenfalls durch ein oder mehrere Heteroatome wie Sauerstoff unterbrochen ist und 5 bis 30 Kohlenstoffatome umfasst, steht;
- **Y** und **Y'**, die gleich oder verschieden sein können, für ein Sauerstoff- oder Schwefelatom stehen; insbesondere Y' für ein Sauerstoffatom steht; und
- **A** für eine aktivierende Gruppe des Kohlenstoffatoms C der (Thio)carbonylgruppe -C(Y)- steht, die in

Gegenwart eines Nucleophils abgehen, A-Y'- freisetzen und eine kovalente Bindung zwischen dem Kohlenstoffatom C der -C(Y)-Gruppe und dem Nucleophil ausbilden kann,

wobei es sich versteht, dass die Zusammensetzungen (A) und (B) zusammen oder separat, vorzugsweise separat, auf die Keratinfasern aufgebracht werden können;
wobei die aktivierte (Thio)esterverbindung bzw. die aktivierten (Thio)esterverbindungen so beschaffen ist bzw. sind, dass die Gruppe **A** für

- eine gegebenenfalls substituierte heterocyclische Gruppe, die über ein Heteroatom wie Stickstoff mit dem Rest des Moleküls verknüpft ist;
- eine gegebenenfalls substituierte Heteroarylgruppe, die über ein Heteroatom wie Stickstoff mit dem Rest des Moleküls verknüpft ist; oder
- eine (Hetero)arylaminogruppe

steht.

**2.** Verfahren nach dem vorhergehenden Anspruch, wobei das nucleophile Alkoxysilan-Polymer oder -Oligomer bzw. die nucleophilen Alkoxysilan-Polymere oder -Oligomere eine oder mehrere nucleophile Gruppen umfasst bzw. umfassen, die aus primären oder sekundären Aminogruppen -N(H)$R_b$ ausgewählt sind, wobei $R_b$ für ein Wasserstoffatom oder eine gegebenenfalls substituierte ($C_1$-$C_6$)Alkylgruppe, eine ($C_5$-$C_{10}$) Cycloalkylgruppe, eine 5- bis 10-gliedrige Heterocycloalkylgruppe oder eine (Hetero)arylgruppe steht, wobei $R_b$ vorzugsweise für ein Wasserstoffatom steht.

**3.** Verfahren nach einem der vorhergehenden Ansprüche, wobei sich das nucleophile Alkoxysilan-Polymer oder -Oligomer bzw. die nucleophilen Alkoxysilan-Polymere oder -Oligomere von einem Monomer der Formel (I) sowie den Säuresalzen davon und den Solvaten davon wie Hydraten ableitet bzw. ableiten, wobei zusammen oder getrennt:

• **p** für 0 steht und **q** für 1 steht;
• $\mathbf{R_1}$ für eine ($C_1$-$C_4$)Alkylgruppe wie Ethyl steht;
• $\mathbf{R_2}$ und $\mathbf{R_3}$ gleich sind und für eine ($C_1$-$C_4$)-Alkoxygruppe wie Ethoxy stehen;
• **ALK** für eine Methylen- oder Propylengruppe steht;
• $\mathbf{X_1}$ für eine Aminogruppe -N($R_a$)- gemäß obiger Definition steht, wobei $R_a$ insbesondere für ein Wasserstoffatom oder eine ($C_1$-$C_4$)Alkylgruppe steht, vorzugsweise $R_a$ für ein Wasserstoffatom steht.

**4.** Verfahren nach einem der vorhergehenden Ansprüche, wobei die aktivierte (Thio)esterverbindung bzw. die aktivierten (Thio)esterverbindungen die nachstehende Formel **(II)** aufweisen, sowie Solvate davon wie Hydrate:

$$\mathbf{R'\text{-}C(Y)\text{-}OA_1} \qquad \mathbf{(II)}$$

wobei in Formel **(II)**:

• **R'** für eine $C_5$-$C_{21}$-, vorzugsweise $C_9$-$C_{17}$- und bevorzugt $C_{11}$-$C_{15}$-Alkylgruppe steht;
• **Y** wie oben definiert ist, vorzugsweise Y für ein Sauerstoffatom steht;
• $\mathbf{A_1}$ für eine reaktive Gruppe steht, die aus $\mathbf{A_{1a}}$, $\mathbf{A_{1b}}$ und $\mathbf{A_{1c}}$ ausgewählt ist:

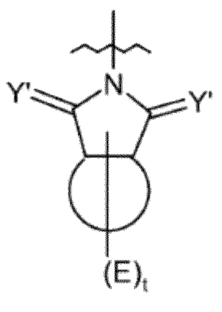

$\mathbf{A_{1a}}$

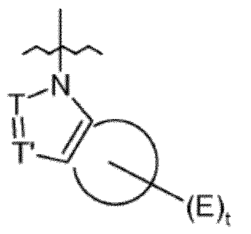

$\mathbf{A_{1b}}$

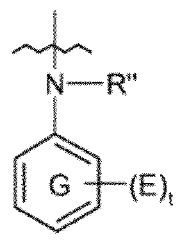

$\mathbf{A_{1c}}$

wobei

o die Variablen **Y'**, die gleich oder verschieden sein können und vorzugsweise gleich sind, für ein Sauerstoff- oder Schwefelatom, vorzugsweise Sauerstoff, stehen;
o für die Bindung steht, die $A_{1a}$, $A_{1b}$ oder $A_{1c}$ mit dem Rest des Moleküls verknüpft;
o **T** und **T'**, die gleich oder verschieden sein können und vorzugsweise identisch sind, für eine Methylengruppe $C(R^a)$, wobei $R^a$ für ein Wasserstoffatom oder ein gegebenenfalls substituiertes $(C_1\text{-}C_6)$Alkyl steht, oder ein Stickstoffatom, insbesondere Stickstoff, stehen oder alternativ dazu T für eine Gruppe $C(R^a)$ wie CH steht und T' für ein Stickstoffatom steht;
o die Variablen **E,** die gleich oder verschieden sein können, wenn t größer oder gleich 2 ist, für ein Atom oder eine elektronenanziehende Gruppe wie Halogen, Nitro, Nitroso, Cyano, Carboxyl, Phosphat, Polyhalogenalkyl, wie Trifluormethyl, Sulfoxy, $SO_3^-M^+$, wobei $M^+$ für ein Wasserstoffatom oder ein kationisches Gegenion wie Alkalimetall, Erdalkalimetall oder Ammonium, wie Natrium oder Kalium, steht, stehen; vorzugsweise E für ein Halogenatom wie Fluor oder Chlor oder eine Gruppe $SO_3^-M^+$ steht;
o **t** für eine ganze Zahl zwischen 0 und 5 inklusive steht;
o **G** für eine Arylgruppe wie Phenyl oder eine Heteroarylgruppe steht;
o , das vorliegen oder fehlen kann, für einen $(C_5\text{-}C_{10})$Cycloalkyl-, $(C_5\text{-}C_{10})$Cycloalkenyl-, 5- bis 10-gliedrigen Heterocycloalkyl-, 5-bis 10-gliedrigen Heterocycloalkenyl-, Arylwie Benzo- oder Heteroaryl-Monocyclus oder anellierten $(C_5\text{-}C_{10})$Cycloalkyl-, $(C_5\text{-}C_{10})$Cycloalkenyl-, 5- bis 10-gliedrigen Heterocycloalkyl-, 5- bis 10-gliedrigen Heterocycloalkenyl-, Aryl- wie Benzo- oder Heteroaryl-Bicyclus, vorzugsweise eine anellierte bicyclische $(C_5\text{-}C_7)$Cycloalkyl-, bicyclische $(C_5\text{-}C_7)$Cycloalkenyl- oder Benzogruppe steht; und
o **R"** für ein Wasserstoffatom oder eine gegebenenfalls substituierte $(C_1\text{-}C_6)$Alkylgruppe wie Benzyl oder eine (Hetero)arylgruppe wie Phenyl steht.

**5.** Verfahren nach dem vorhergehenden Anspruch, wobei die aktivierte (Thio)esterverbindung bzw. die aktivierten (Thio)esterverbindungen der Formel **(II)** sowie die Solvate davon wie Hydrate so beschaffen sind, dass $\mathbf{A_1}$ aus $\mathbf{A'_{1a}}$, $\mathbf{A''_{1a}}$, $\mathbf{A'''_{1a}}$, $\mathbf{A''''_{1a}}$, $\mathbf{A'_{1b}}$, $\mathbf{A''_{1b}}$ und $\mathbf{A'_{1c}}$ ausgewählt ist:

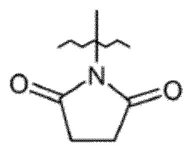
$\mathbf{A'_{1a}}$

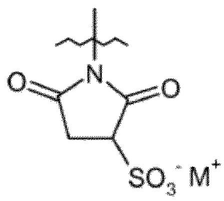
$\mathbf{A''_{1a}}$

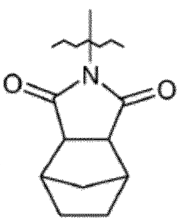
$\mathbf{A'''_{1a}}$

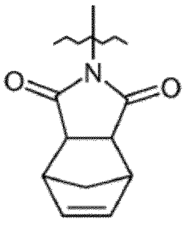
$\mathbf{A''''_{1a}}$

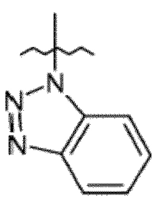
$\mathbf{A'_{1b}}$

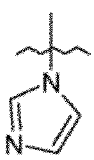
$\mathbf{A''_{1b}}$

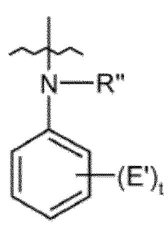
$\mathbf{A'_{1c}}$

wobei:

- $\mathbf{M^+}$ für ein kationisches Gegenion gemäß obiger Definition und insbesondere für ein Alkalimetall wie Natrium steht;
- die Variablen **E'**, die gleich oder verschieden sein können, wenn t größer gleich 2 ist, für eine elektronenanziehende Gruppe wie ein Halogenatom, das insbesondere aus Fluor und Chlor ausgewählt ist, stehen;
- **t** für eine ganze Zahl zwischen 0 und 5 inklusive steht;
- **R"** wie oben definiert ist und insbesondere für ein Wasserstoffatom oder eine $(C_1\text{-}C_6)$Alkylgruppe, vorzugsweise

ein Wasserstoffatom, steht;

vorzugsweise $\mathbf{A_1}$ für eine Gruppe $\mathbf{A'_{1a}}$, $\mathbf{A''_{1a}}$ oder $\mathbf{A''_{1b}}$, vorzugsweise $\mathbf{A'_{1a}}$, steht.

**6.** Verfahren nach einem der Ansprüche 4 und 5, wobei die aktivierte (Thio)esterverbindung bzw. die aktivierten (Thio)esterverbindungen der Formel **(II)** so beschaffen ist bzw. sind, dass $\mathbf{A_1}$ für eine Gruppe $\mathbf{A'_{1a}}$ oder $\mathbf{A''_{1a}}$, vorzugsweise $\mathbf{A'_{1a}}$, steht und **R'** für eine $C_9$-$C_{17}$- und bevorzugt $C_{11}$-$C_{15}$-Alkylgruppe steht.

**7.** Verfahren nach einem der vorhergehenden Ansprüche, wobei die aktivierte (Thio)esterverbindung bzw. die aktivierten (Thio)esterverbindungen aus Estern von Laurinsäure, Palmitinsäure, Pentansäure, Hexansäure, 2-Ethylhexansäure, Octansäure, Nonansäure, Decansäure, Dodecansäure, Hexadecansäure, Eicosansäure, Hexacosansäure, Octadecensäure, Undecensäure, Eicosatetraensäure oder Octadecatriensäure und von *N*-Hydroxysuccinimid ausgewählt ist bzw. sind.

**8.** Verfahren nach einem der vorhergehenden Ansprüche, wobei die aktivierte (Thio)esterverbindung bzw. die aktivierten (Thio)esterverbindungen in einem Gehalt im Bereich von 0,1 bis 5 Gew.-%, bezogen auf das Gesamtgewicht der Zusammensetzung, in der die aktivierten (Thio)esterverbindungen enthalten sind, vorzugsweise im Bereich von 0,5 bis 4 Gew.-% und bevorzugt im Bereich von 1 bis 4 Gew.-% vorliegt bzw. vorliegen.

**9.** Verfahren nach einem der vorhergehenden Ansprüche, wobei die Zusammensetzungen (A) und (B) zusammen auf die Keratinfasern aufgebracht werden, die Zusammensetzungen (A) und (B) vor dem Aufbringen zu einer Zusammensetzung (C) gemischt werden können, wobei die Zusammensetzung (C) dann auf die Keratinfasern aufgebracht wird.

**10.** Verfahren nach einem der Ansprüche 1 bis 8, wobei Zusammensetzung (B) zuerst aufgebracht wird und anschließend Zusammensetzung (A) aufgebracht wird; vorzugsweise zwischen dem Schritt des Aufbringens von Zusammensetzung (B) und Zusammensetzung (A) keine Zwischenspülung mit Wasser durchgeführt wird.

**11.** Verfahren nach einem der Ansprüche 1 bis 8, wobei Zusammensetzung (A) zuerst aufgebracht wird und anschließend Zusammensetzung (B) aufgebracht wird; vorzugsweise zwischen dem Schritt des Aufbringens von Zusammensetzung (A) und Zusammensetzung (B) keine Zwischenspülung mit Wasser durchgeführt wird.

**12.** Verfahren nach einem der Ansprüche 10 oder 11, wobei zwischen dem Schritt des Aufbringens der Zusammensetzung von Schritt (B) und der Zusammensetzung (A) oder zwischen dem Schritt des Aufbringens der Zusammensetzung (A) und der Zusammensetzung (B) ein Schritt zum Trocknen der Keratinfasern durchgeführt wird; der Trocknungsschritt vorzugsweise vollständig ist und bei Raumtemperatur oder durch Wärmebehandlung, vorzugsweise bei Raumtemperatur, durchgeführt werden kann; die Wärmebehandlung bei einer Temperatur zwischen 30 und 250 °C, insbesondere mit einer Trockenhaube, einem Haartrockner, einem Lockenstab oder Glätteisen, einem Infrarotabgabegerät oder anderen Heizvorrichtungen und in Kunststofffolie, vorzugsweise mit einem Haushaltshaartrockner, insbesondere bei einer Temperatur von 50 °C und 100 °C, durchgeführt wird.

**13.** Verfahren nach einem der vorhergehenden Ansprüche, wobei nach dem Aufbringen der kosmetischen Zusammensetzung (A), (B) oder (C) auf die Keratinfasern die aufgebrachte Zusammensetzung über einen Zeitraum im Bereich von 1 bis 60 Minuten, vorzugsweise im Bereich von 2 bis 50 Minuten, bevorzugt im Bereich von 5 bis 30 Minuten, auf die Fasern einwirken gelassen wird; die Einwirkungszeit bei einer Temperatur im Bereich von 15 °C bis 45 °C, vorzugsweise bei Raumtemperatur (25 °C) erfolgen kann; die Keratinfasern vorzugsweise nach der Behandlung mit Wasser gespült oder mit einem Shampoo gewaschen werden, die Keratinfasern dann gegebenenfalls mit einem Haartrockner oder einer Haube oder an der Luft getrocknet werden; das Verfahren insbesondere vor, während und/oder nach einem zusätzlichen Verfahren zur kosmetischen Behandlung der Keratinfasern, wie einem Verfahren zur temporären Formgebung, insbesondere Formgebung mit Lockenwicklern, einem Kräuseleisen oder einem Glätteisen, oder einem Verfahren zur lang anhaltenden Formgebung, insbesondere Dauerwellbehandlung oder Entkräuselung der Keratinfasern, durchgeführt werden kann.

**14.** Kosmetische Zusammensetzung, umfassend:

i) mindestens ein nucleophiles Alkoxysilan-Polymer gemäß einem der Ansprüche 1 bis 3 und
ii) mindestens eine aktivierte (Thio)esterverbindung der Formel **R-C(Y)-Y'-A,** wobei:

- **R** für eine lineare oder verzweigte, gesättigte oder ungesättigte, vorzugsweise gesättigte, gegebenenfalls substituierte, vorzugsweise unsubstituierte, aliphatische Kette auf Kohlenwasserstoffbasis, die gegebenenfalls durch ein oder mehrere Heteroatome wie Sauerstoff unterbrochen ist und 5 bis 30 Kohlenstoffatome umfasst, steht;
- **Y** und **Y',** die gleich oder verschieden sein können, für ein Sauerstoff- oder Schwefelatom stehen; insbesondere Y' für ein Sauerstoffatom steht; und
- **A** für eine aktivierende Gruppe des Kohlenstoffatoms C der (Thio)carbonylgruppe -C(Y)- steht, die in Gegenwart eines Nucleophils abgehen, A-Y'- freisetzen und eine kovalente Bindung zwischen dem Kohlenstoffatom C der -C(Y)-Gruppe und dem Nucleophil ausbilden kann,

wobei die aktivierte (Thio)esterverbindung bzw. die aktivierten (Thio)esterverbindungen so beschaffen ist bzw. sind, dass die Gruppe **A** für

- eine gegebenenfalls substituierte heterocyclische Gruppe, die über ein Heteroatom wie Stickstoff mit dem Rest des Moleküls verknüpft ist;
- eine gegebenenfalls substituierte Heteroarylgruppe, die über ein Heteroatom wie Stickstoff mit dem Rest des Moleküls verknüpft ist; oder
- eine (Hetero)arylaminogruppe

steht.

**15.** Vorrichtung mit mehreren Kompartimenten oder Kit, umfassend in einem ersten Kompartiment:

i) mindestens ein nucleophiles Alkoxysilan-Polymer gemäß einem der Ansprüche 1 bis 3 und
in einem zweiten Kompartiment:
ii) mindestens eine aktivierte (Thio)esterverbindung der Formel **R-C(Y)-Y'-A,** wobei:

- **R** für eine lineare oder verzweigte, gesättigte oder ungesättigte, vorzugsweise gesättigte, gegebenenfalls substituierte, vorzugsweise unsubstituierte, aliphatische Kette auf Kohlenwasserstoffbasis, die gegebenenfalls durch ein oder mehrere Heteroatome wie Sauerstoff unterbrochen ist und 5 bis 30 Kohlenstoffatome umfasst, steht;
- **Y** und **Y'**, die gleich oder verschieden sein können, für ein Sauerstoff- oder Schwefelatom stehen; insbesondere Y' für ein Sauerstoffatom steht; und
- **A** für eine aktivierende Gruppe des Kohlenstoffatoms C der (Thio)carbonylgruppe -C(Y)- steht, die in Gegenwart eines Nucleophils abgehen, A-Y'- freisetzen und eine kovalente Bindung zwischen dem Kohlenstoffatom C der -C(Y)-Gruppe und dem Nucleophil ausbilden kann,
wobei die aktivierte (Thio)esterverbindung bzw. die aktivierten (Thio)esterverbindungen so beschaffen ist bzw. sind, dass die Gruppe **A** für
- eine gegebenenfalls substituierte heterocyclische Gruppe, die über ein Heteroatom wie Stickstoff mit dem Rest des Moleküls verknüpft ist;
- eine gegebenenfalls substituierte Heteroarylgruppe, die über ein Heteroatom wie Stickstoff mit dem Rest des Moleküls verknüpft ist; oder
- eine (Hetero)arylaminogruppe

steht;
wobei die Bestandteile i) und ii) jeweils in einer separaten Verpackungsanordnung verpackt sind.

## Revendications

**1.** Procédé pour le traitement de fibres de kératine, notamment de fibres de kératine humaines telles que les cheveux, comprenant :

i) l'application aux fibres de kératine d'une composition cosmétique (A) comprenant au moins un polymère ou oligomère d'alcoxysilane nucléophile, dans laquelle le(s) polymère(s) ou l'oligomère d'alcoxysilane nucléophile est/sont issu(s) d'un monomère de formule **(I)** ci-dessous, et aussi des sels d'acide correspondants, et des solvates correspondants tels que des hydrates :

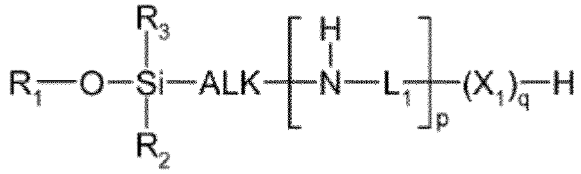

(I)

dans laquelle formule **(I)** :

• **p** et **q**, qui peuvent être identiques ou différents, sont égaux à 0 ou 1, la somme p + q étant supérieure ou égale à 1, préférablement p + q est égale à 1 ou 2, en particulier p est 0 et q est 1 ;
• $\mathbf{X_1}$ représente un groupe amino $-N(R_a)-$, $R_a$ représentant un atome d'hydrogène ou un groupe choisi parmi $C_1$-$C_8$-alkyle tel que méthyle, $C_5$-$C_7$-cycloalkyle tel que cyclohexyle, aryle tel que phényle et aryl-$C_1$-$C_4$-alkyle tel que benzyle, préférablement -N(H)- ;
• **ALK** représente une chaîne à base d'hydrocarbure divalente saturée en $C_1$-$C_{10}$ linéaire ou ramifiée, en particulier linéaire, plus particulièrement $-(CH_2)_x-$, x représentant un entier compris entre 1 et 6 inclus, préférablement entre 1 et 4 telle que méthylène, éthylène ou propylène ; plus préférentiellement méthylène ou propylène ;
• $\mathbf{R_1}$ représente un atome d'hydrogène ou un groupe $C_1$-$C_6$-alkyle tel que méthyle ou éthyle ;
• $\mathbf{R_2}$ et $\mathbf{R_3}$, qui peuvent être identiques ou différents, préférablement identiques, représentent un groupe choisi parmi hydroxyle, $C_1$-$C_6$-alkyle et $C_1$-$C_6$-alcoxy, en particulier $C_1$-$C_4$-alcoxy tel qu'éthoxy ;
• $\mathbf{L_1}$ représente une chaîne à base d'hydrocarbure divalente en $C_{1-}C_{20}$ saturée linéaire ou ramifiée ; en particulier linéaire, plus particulièrement $-(CH_2)_x-$, x étant tel que défini précédemment ; en particulier, $L_1$ représente un groupe éthylène ou propylène ; et

ii) l'application d'une composition cosmétique (B) comprenant au moins un composé (thio)ester activé de formule **R-C(Y)-Y'-A** :

- **R** représentant une chaîne aliphatique à base d'hydrocarbure linéaire ou ramifiée, saturée ou insaturée, préférablement saturée, éventuellement substituée, préférablement non substituée, éventuellement interrompue par un ou plusieurs hétéroatomes tels qu'oxygène, comprenant de 5 à 30 atomes de carbone ;
- **Y** et **Y'**, qui peuvent être identiques ou différents, représentent un atome d'oxygène ou de soufre ; en particulier, Y' représente un atome d'oxygène ; et
- **A** représentant un groupe d'activation de l'atome de carbone C du groupe (thio)carbonyle -C(Y)-, qui est capable de partir en présence d'un nucléophile, de libérer A-Y'- et de créer une liaison covalente entre l'atome de carbone C du groupe -C(Y)- et ledit nucléophile,

étant entendu que les compositions (A) et (B) peuvent être appliquées aux fibres de kératine ensemble ou séparément, préférablement séparément ;
dans lequel le(s) composé(s) (thio)ester(s) activé(s) est/sont tel(s) que le groupe **A** est :

- un groupe hétérocyclique éventuellement substitué relié au reste de la molécule via un hétéroatome tel qu'azote ;
- un groupe hétéroaryle éventuellement substitué relié au reste de la molécule via un hétéroatome tel qu'azote ; ou
- un groupe (hétéro)arylamino.

**2.** Procédé selon la revendication précédente, dans lequel le (s) polymère (s) ou l'oligomère d'alcoxysilane nucléophile comprend/comprennent un ou plusieurs groupes nucléophiles choisis parmi des groupes amino primaires et secondaires $-N(H)R_b$, $R_b$ représentant un atome d'hydrogène ou un groupe $C_1$-$C_6$-alkyle éventuellement substitué, un groupe $C_5$-$C_{10}$-cycloalkyle, un groupe hétérocycloalkyle à 5 à 10 chaînons ou un groupe (hétéro) aryle, préférablement $R_b$ représentant un atome d'hydrogène.

**3.** Procédé selon l'une quelconque des revendications précédentes, dans lequel le(s) polymère(s) ou l'oligomère d'alcoxysilane nucléophile issu(s) d'un monomère de formule (I) est/sont, et aussi les sels d'acide correspondants, et les solvates correspondants tels que les hydrates, pris ensemble ou séparément :

- **p** est 0 et **q** est 1 ;
- **$R_1$** représente un groupe $C_1$-$C_4$-alkyle tel qu'éthyle ;
- **$R_2$** et **$R_3$** sont identiques et représentent un groupe $C_1$-$C_4$-alcoxy tel qu'éthoxy ;
- **ALK** représente un groupe méthylène ou propylène ;
- **$X_1$** représente un groupe amino -N($R_a$)- tel que défini précédemment, en particulier, $R_a$ représentant un atome d'hydrogène ou un groupe $C_1$-$C_4$-alkyle ; préférablement, $R_a$ représentant un atome d'hydrogène.

**4.** Procédé selon l'une quelconque des revendications précédentes, dans lequel le(s) composé(s) (thio)ester(s) activé(s) est/sont de formule **(II)** ci-dessous, et aussi les solvates correspondants tels que les hydrates :

**R'-C(Y)-OA$_1$** **(II)**

dans laquelle formule **(II)** :

- **R'** représente un groupe alkyle en $C_5$-$C_{21}$, préférablement en $C_9$-$C_{17}$ et préférentiellement en $C_{11}$-$C_{15}$ ;
- **Y** est tel que défini précédemment ; préférablement, Y représente un atome d'oxygène ;
- **$A_1$** désigne un groupe réactif choisi parmi **$A_{1a}$**, **$A_{1b}$** et **$A_{1c}$** :

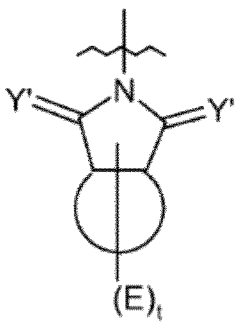

**$A_{1a}$**

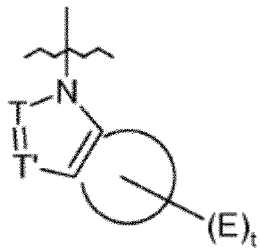

**$A_{1b}$**

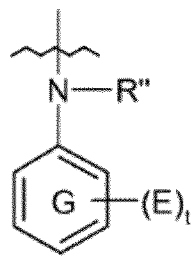

**$A_{1c}$**

◦ les **Y'**, qui peuvent être identiques ou différents, préférablement identiques, représentant un atome d'oxygène ou de soufre, préférablement oxygène ;

◦ 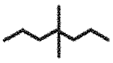 représentant la liaison qui relie $A_{1a}$, $A_{1b}$ ou $A_{1c}$ au reste de la molécule ;

◦ **T** et **T'**, qui peuvent être identiques ou différents, préférablement identiques, représentant un groupe méthylène C($R^a$), $R^a$ représentant un atome d'hydrogène ou un $C_1$-$C_6$-alkyle éventuellement substitué, ou un atome d'azote, en particulier azote, ou en variante T représente un groupe C($R^a$) tel que CH et T' représente un atome d'azote ;

o les **E,** qui peuvent être identiques ou différents, lorsque t est supérieur ou égal à 2, représentant un atome ou un groupe électroattracteur tel qu'halogène, nitro, nitroso, cyano, carboxyle, phosphate, polyhalogénoalkyle, tel que trifluorométhyle, sulfoxy, $SO_3^-M^+$, $M^+$ représentant un atome d'hydrogène, ou un contre-ion cationique tel qu'un métal alcalin, un métal alcalino-terreux ou un ammonium, tel que sodium ou potassium ; préférablement, E représente un atome d'halogène tel que fluor ou chlore ou un groupe $SO_3^-M^+$ ;

◦ **t** représentant un entier compris entre 0 et 5 inclus ;

◦ **G** représentant un groupe aryle tel que phényle ou un groupe hétéroaryle ;

◦

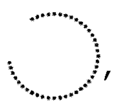 ,

qui peut être présent ou absent, représente un monocycle $C_5$-$C_{10}$-cycloalkyle, $C_5$-$C_{10}$-cycloalcényle, hétérocycloalkyle à 5 à 10 chaînons, hétérocycloalcényle à 5 à 10 chaînons, aryle tel que benzo ou hétéroaryle ou un bicycle condensé ; préférablement un groupe $C_5$-$C_7$-cycloalkyle bicyclique condensé, $C_5$-$C_7$-cycloal-

cényle bicyclique ou benzo ; et

◦ **R"** représentant un atome d'hydrogène ou un groupe $C_1$-$C_6$-alkyle éventuellement substitué tel que benzyle ou un groupe (hétéro)aryle tel que phényle.

5. Procédé selon la revendication précédente, dans lequel le(s) composé(s) (thio)ester(s) activé(s) de formule **(II)**, et aussi les solvates correspondants tels que les hydrates, sont tels que $\mathbf{A_1}$ est choisi parmi $\mathbf{A'_{1a}}$, $\mathbf{A''_{1a}}$, $\mathbf{A'''_{1a}}$, $\mathbf{A''''_{1a}}$, $\mathbf{A'_{1b}}$, $\mathbf{A''_{1b}}$ et $\mathbf{A''_{1c}}$ :

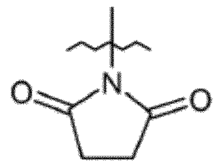

$\mathbf{A'_{1a}}$

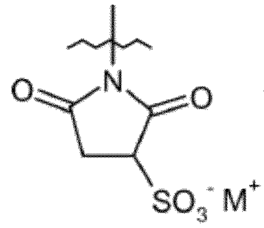

$\mathbf{A''_{1a}}$

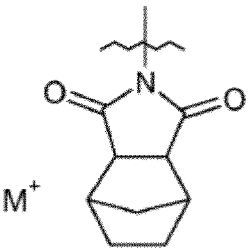

$\mathbf{A'''_{1a}}$

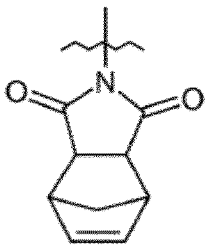

$\mathbf{A''''_{1a}}$

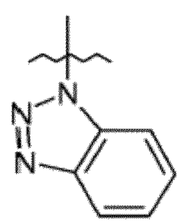

$\mathbf{A'_{1b}}$

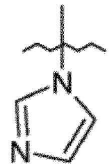

$\mathbf{A''_{1b}}$

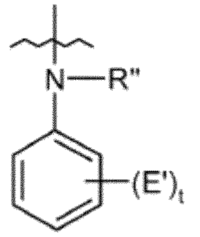

$\mathbf{A'_{1c}}$

- $\mathbf{M^+}$ représentant un contre-ion cationique tel que défini précédemment, en particulier représentant un métal alcalin tel que sodium ;
- les **E',** qui peuvent être identiques ou différents, lorsque t est supérieur ou égal à 2, représentant un groupe électroattracteur tel qu'un atome d'halogène, choisi en particulier parmi fluor et chlore ;
- **t** est un entier compris entre 0 et 5 inclus ;
- **R"** étant tel que défini précédemment, en particulier représentant un atome d'hydrogène ou un groupe $C_1$-$C_6$-alkyle, préférablement un atome d'hydrogène ;

préférablement, $\mathbf{A_1}$ représente un groupe $\mathbf{A'_{1a}}$, $\mathbf{A''_{1a}}$ ou $\mathbf{A''_{1b}}$, préférablement $\mathbf{A'_{1a}}$.

6. Procédé selon l'une ou l'autre des revendications 4 et 5, dans lequel le(s) composé(s) (thio)ester(s) activé(s) de formule **(II)** est/sont tel(s) que $\mathbf{A_1}$ représente un groupe $\mathbf{A'_{1a}}$ ou $\mathbf{A''_{1a}}$, préférablement $\mathbf{A'_{1a}}$ et **R'** représente un groupe alkyle en $C_9$-$C_{17}$ et préférentiellement en $C_{11}$-$C_{15}$.

7. Procédé selon l'une quelconque des revendications précédentes, dans lequel le(s) composé(s) (thio)ester(s) activé(s) est/sont choisi(s) parmi : les esters d'acide laurique, palmitique, pentanoïque, hexanoïque, 2-éthylhexanoïque, octanoïque, nonanoïque, décanoïque, dodécanoïque, hexadécanoïque, éicosanoïque, hexacosanoïque, octadécénoïque, undécénoïque, éicosatétraénoïque ou octadécatriénoïque et de N-hydroxysuccinimide.

8. Procédé selon l'une quelconque des revendications précédentes, dans lequel le(s) composé(s) (thio)ester(s) activé(s) est/sont présent(s) en une teneur allant de 0,1 % à 5 % en poids, par rapport au poids total de la composition dans laquelle lesdits composés (thio)esters activés sont contenus, préférablement allant de 0,5 % à 4 % en poids, et préférentiellement allant de 1 % à 4 % en poids.

9. Procédé selon l'une quelconque des revendications précédentes, dans lequel les compositions (A) et (B) sont appliquées ensemble aux fibres de kératine, les compositions (A) et (B) peuvent être mélangées ensemble avant

l'application pour donner une composition (C), ladite composition (C) étant ensuite appliquée aux fibres de kératine.

**10.** Procédé selon l'une quelconque des revendications 1 à 8, dans lequel la composition (B) est appliquée en premier et la composition (A) est appliquée subséquemment ; préférablement, entre l'étape d'application de la composition (B) et de la composition (A), aucun rinçage intermédiaire avec de l'eau n'est réalisé.

**11.** Procédé selon l'une quelconque des revendications 1 à 8, dans lequel la composition (A) est appliquée en premier et la composition (B) est appliquée subséquemment ; préférablement, entre l'étape d'application de la composition (A) et de la composition (B), aucun rinçage intermédiaire avec de l'eau n'est réalisé.

**12.** Procédé selon l'une quelconque des revendications 10 ou 11 dans lequel entre l'étape d'application de la composition (B) et de la composition (A) ou entre l'étape d'application de la composition (A) et de la composition (B), une étape pour le séchage des fibres de kératine est mise en œuvre ; l'étape de séchage est préférablement terminée, elle peut être réalisée à température ambiante ou par un traitement à la chaleur, préférablement à température ambiante ; ledit traitement à la chaleur est réalisé à une température comprise entre 30 et 250 °C notamment avec un casque capillaire, un sèche-cheveux, un fer à friser ou un fer plat, un diffuseur d'infrarouges ou d'autres dispositifs de chauffage, et dans un film de plastique, préférablement avec un sèche-cheveux domestique notamment à une température de 50 °C et 100 °C.

**13.** Procédé selon l'une quelconque des revendications précédentes, dans lequel, après l'application de la composition cosmétique (A), (B) ou (C) aux fibres de kératine, ladite composition appliquée est laissée reposer sur les fibres pendant un temps allant de 1 à 60 minutes, préférablement allant de 2 à 50 minutes, préférentiellement allant de 5 à 30 minutes ; le temps de pose peut avoir lieu à une température allant de 15 °C à 45 °C, préférablement à température ambiante (25 °C) ; préférablement, après le traitement, les fibres de kératine sont rincées avec de l'eau ou lavées avec un shampooing, les fibres de kératine sont ensuite éventuellement séchées avec un sèche-cheveux ou un casque ou à l'air libre ; en particulier, le procédé peut être réalisé avant, pendant et/ou après un procédé supplémentaire de traitement cosmétique des fibres de kératine, tel qu'un procédé de mise en forme temporaire, notamment une mise en forme avec des bigoudis, un fer à gaufrer ou un fer à lisser, ou un procédé de mise en forme durable, notamment une permanente ou une relaxation des fibres de kératine.

**14.** Composition cosmétique comprenant :

i) au moins un polymère d'alcoxysilane nucléophile tel que défini dans l'une quelconque des revendications 1 à 3 ; et
ii) au moins un composé (thio)ester activé de formule **R-C(Y)-Y'-A** :

- **R** représentant une chaîne aliphatique à base d'hydrocarbure linéaire ou ramifiée, saturée ou insaturée, préférablement saturée, non substituée, éventuellement interrompue par un ou plusieurs hétéroatomes tels qu'oxygène, comprenant de 5 à 30 atomes de carbone ;
- **Y** et **Y',** qui peuvent être identiques ou différents, représentent un atome d'oxygène ou de soufre ; en particulier, Y' représente un atome d'oxygène ; et
- **A** représentant un groupe d'activation de l'atome de carbone C du groupe (thio)carbonyle -C(Y)-, qui est capable de partir en présence d'un nucléophile, de libérer A-Y'- et de créer une liaison covalente entre l'atome de carbone C du groupe -C(Y)- et ledit nucléophile ;

dans laquelle le(s) composé(s) (thio)ester(s) activé(s) est/sont tel(s) que le groupe **A** est :

- un groupe hétérocyclique éventuellement substitué relié au reste de la molécule via un hétéroatome tel qu'azote ;
- un groupe hétéroaryle éventuellement substitué relié au reste de la molécule via un hétéroatome tel qu'azote ; ou
- un groupe (hétéro)arylamino.

**15.** Dispositif ou kit à plusieurs compartiments, comprenant, dans un premier compartiment :

i) au moins un polymère d'alcoxysilane nucléophile tel que défini dans l'une quelconque des revendications 1 à 3 ; et

dans un second compartiment :
ii) au moins un composé (thio)ester activé de formule **R-C(Y)-Y'-A** :

- **R** représentant une chaîne aliphatique à base d'hydrocarbure linéaire ou ramifiée, saturée ou insaturée, préférablement saturée, non substituée, éventuellement interrompue par un ou plusieurs hétéroatomes tels qu'oxygène, comprenant de 5 à 30 atomes de carbone ;
- **Y** et **Y'**, qui peuvent être identiques ou différents, représentent un atome d'oxygène ou de soufre ; en particulier, Y' représente un atome d'oxygène ; et
- **A** représentant un groupe d'activation de l'atome de carbone C du groupe (thio)carbonyle -C(Y)-, qui est capable de partir en présence d'un nucléophile, de libérer A-Y'- et de créer une liaison covalente entre l'atome de carbone C du groupe -C(Y)- et ledit nucléophile ;

dans lequel le(s) composé(s) (thio)ester(s) activé(s) est/sont tel(s) que le groupe **A** est :

- un groupe hétérocyclique éventuellement substitué relié au reste de la molécule via un hétéroatome tel qu'azote ;
- un groupe hétéroaryle éventuellement substitué relié au reste de la molécule via un hétéroatome tel qu'azote ; ou
- un groupe (hétéro)arylamino ;

les ingrédients i) et ii) étant chacun emballés dans un assemblage d'emballages séparés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008156327 A
- WO 20081563 A
- KR 20120036877
- US 4344763 A
- US 2009293899 A
- JP 2012240983 A
- FR 2982155


### Non-patent literature cited in the description


- Evaluation of product efficacy. **C. BOUILLON ; J. WILKINSON.** The Science of Hair Care. 2005, 407